# EUROPÄISCHE PATENTSCHRIFT

(11) **EP 3 601 209 B1**
(45) Veröffentlichungstag und Bekanntmachung des Hinweises auf die Patenterteilung: **27.11.2024**
(21) Anmeldenummer: 18724128.6
(22) Anmeldetag: 26.03.2018
(51) Int. Cl.: C07C 69/67, C07C 69/708, C07C 69/73, C07C 69/75, C07C 69/80, C07C 323/52, C10M 105/42

(54) **NEUE ESTERVERBINDUNGEN, VERFAHREN ZU IHRER HERSTELLUNG SOWIE IHRE VERWENDUNG**
NOVEL ESTER COMPOUNDS, METHOD FOR THE PRODUCTION THEREOF AND USE THEREOF
NOUVEAUX COMPOSÉS ESTERS, PROCÉDÉ DE PRODUCTION DESDITS COMPOSÉS ET LEUR UTILISATION

(30) Priorität: 29.03.2017 DE 102017003040; 14.03.2018 DE 102018002041
(43) Veröffentlichungstag der Anmeldung: 05.02.2020
(73) Patentinhaber: Klüber Lubrication München GmbH & Co. KG, 81379 München (DE)
(72) Erfinder: SEEMEYER, Stefan, 81379 München (DE); ERHARD, Maximilian, 81379 München (DE); KILTHAU, Thomas, 81379 München (DE); MA, Ling, 81379 München (DE)
(74) Vertreter: Puschmann Borchert Kaiser Klettner Patentanwälte Partnerschaft mbB
(86) Internationale Anmeldenummer: PCT/EP2018/000117
(87) Internationale Veröffentlichungsnummer: WO 2018/177588

(56) Entgegenhaltungen:
- WO-A1-2012/173666
- WO-A1-2014/078149
- US-A1- 2013 261 325
- US-A1- 2016 108 343
- ARUKALI SAMMAIAH ET AL: "Synthesis and physical properties of novel estolides from dicarboxylic acids and methyl ricinoleate : Synthesis and physical properties of novel estolides", EUROPEAN JOURNAL OF LIPID SCIENCE AND TECHNOLOGY., vol. 118, no. 3, 1 March 2016 (2016-03-01), DE, pages 486 - 494, XP055491355, ISSN: 1438-7697, DOI: 10.1002/ejlt.201500109
- XU XU ET AL: "Enhanced thermal and mechanical properties of lignin/polypropylene wood-plastic composite by using flexible segment-containing reactive compatibilizer", MACROMOLECULAR RESEARCH, vol. 22, no. 10, 24 September 2014 (2014-09-24), KR, pages 1084 - 1089, XP055491487, ISSN: 1598-5032, DOI: 10.1007/s13233-014-2161-3
- GORLA GEETHANJALI ET AL: "Synthesis, Characterization, and Evaluation of Castor Oil-Based Acylated Derivatives as Potential Lubricant Base Stocks", INDUSTRIAL & ENGINEERING CHEMISTRY RESEARCH, vol. 55, no. 34, 22 August 2016 (2016-08-22), pages 9109 - 9117, XP055491373, ISSN: 0888-5885, DOI: 10.1021/acs.iecr.6b01550

## Beschreibung

Die Erfindung betrifft neue Esterverbindungen auf der Basis von Di-, Tri- und höher funktionellen Carbonsäuren gemäß der allgemeinen Formel (I)

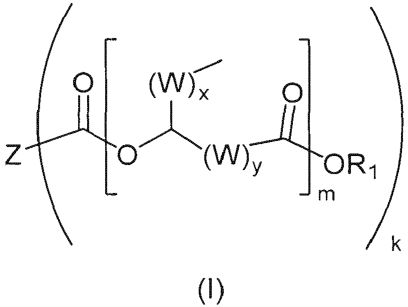

Verfahren zu ihrer Herstellung sowie ihre Verwendung in Schmierstoffen.

Esterverbindungen sind in den letzten Jahren immer häufiger in Schmiermittelzubereitungen eingesetzt worden. Bei den bekannten Esterverbindungen werden bei Anwendung des Schmiermittels in Gegenwart von Wasser der Ester in die Fettsäure und den Alkohol gespalten. Diese Reaktion kann beispielsweise durch Säuren, Basen oder durch Kupfer katalysiert werden. Das hat die Zerstörung der Moleküle zur Folge. Dementsprechend verlieren die Schmierstoffe an Schmierwirkung. Daher besteht ein hoher Bedarf an hydrolysestabilen Estern.

Des weiteren sind herkömmliche Schmierstoffe beispielsweise für Hochtemperaturanwendungen nicht geeignet, da sie bei hohen Temperaturen durch Oxidations- und/oder thermische Zersetzungsvorgänge sowie durch Polymerisationen zerstört werden können und dadurch ihre schmierenden Eigenschaften und Wirkungen stark eingeschränkt werden. Bei Zersetzungsreaktionen wird der Schmierstoff in niedermolekulare flüchtige Komponenten gespalten. Das Verdampfen dieser flüchtigen Komponenten führt zu unerwünschten Viskositätsänderungen, Ölverlust und zur übermäßigen Dampfbildung. Hieraus resultiert ebenfalls ein Verlust der Schmierwirkung. Auch durch Polymerisation verlieren die Schmierstoffe aufgrund der Bildung unlöslicher Polymerisationsprodukte ihre Schmierwirkung. Diese Verschmutzungen müssen entfernt werden, wodurch sich die Wartungsarbeiten erhöhen. Es werden darüber hinaus chemische Abfallstoffe produziert, die aufwendig entsorgt werden müssen. Aufgrund der vermehrten Reinigungs- und Wartungsarbeiten erhöhen sich die Ausfallzeiten der zu schmierenden Vorrichtungen. Insgesamt führt die Verwendung von ungeeigneten Schmierstoffen bei Hochtemperaturanwendungen zu höheren Kosten, da die Arbeitsgeräte verschmutzen und ein höherer Bedarf an Schmierstoffen besteht. Darüber hinaus sinkt die Produktqualität.

Um den vielfältigen Anforderungen gerecht zu werden, müssen Schmierstoffe unter anderem eine hohe Stabilität, niedrige Reibungsbeiwerte und hohe Verschleißfestigkeiten aufweisen.

Hohe Temperaturen treten oftmals bei der Verwendung in Ketten, Wälz- und Gleitlagern, in der Fahrzeugtechnik, der Fördertechnik, dem Maschinenbau, der Bürotechnik sowie in industriellen Anlagen und Maschinen, aber auch in den Bereichen der Haushaltsmaschinen und der Unterhaltungselektronik, auf.

Hohe Verarbeitungstemperaturen treten oftmals bei der Lebensmittelverarbeitung auf, wie beim Kochen, Backen, Sieden, Rösten, Schmoren, Sterilisieren, Braten und Dämpfen. Bei diesen Vorgängen kommen diverse Arbeitsgeräte zum Einsatz. Zur Schmierung dieser Arbeitsgeräte sind hochtemperaturbeständige Schmierstoffe notwendig.

An die Basisöle zum Schmieren von Arbeitsgeräten für die Verarbeitung von Lebensmitteln werden besondere Anforderungen in Bezug auf ihre Umweltverträglichkeit und Toxizität gestellt. Grundsätzlich sollte ein lebensmittelverträglicher Schmierstoff H1 tauglich sein, wenn der Schmierstoff mittelbar oder unmittelbar mit Nahrungs-, Genuss- und Lebensmitteln in Kontakt kommen kann. Zu den bevorzugten Anwendungsbereichen in der Lebensmittelindustrie gehören Ketten in Backöfen und anderen Hochtemperaturanwendungen, sowie Transportgehänge, insbesondere Trolleys und deren Lager.

Diese Schmierstoffe unterliegen gesetzlichen Vorschriften, wie der Zertifizierung nach NSF/H1 oder NSF/H2.

Bei Anwendungen von Schmierstoffen im Marinebereich, die sich zumeist unterhalb der Wasserlinie befinden, besteht das Risiko, die Meeres- bzw. Gewässerumwelt durch Austritt von Schmierstoffen zu kontaminieren. Obwohl versucht wird, bei diesen Anwendungen die Wasserseite bestmöglich abzudichten, sind Schmierstoffverluste alltäglich. Laut einer Quelle der "United States Environmental Protection Agency" im Jahre 2011 verlieren unterschiedliche Schiffsbauten von weniger als einen Liter Schmierstoff bis hin zu 20 Liter pro Tag und Schiff.

Die US 2016/108343 A1 beschreibt bespielsweise Estolide, die in Schmiermitteln eingesetzt werden. Diese Estolide werden in nicht-oligomerer Form hergestellt, wobei als Linkerverbindungen nur Di- und höhere Carbonsäuren eingesetzt werden. Die erhaltenen Schmiermittel weisen geringe Hydrolyseständigkeiten und einen geringen Viskositätsindex auf.

Die bisher bekannten Schmierstoffe, können aber allen diesen Anforderungen nicht genügen.

Ester, die höher funktionelle Carbonsäuren als zentrales Molekül enthalten, wie z.B. Dimersäure sind bekannt. Diese Verbindungen sind aber in der Regel nicht biologisch abbaubar. Vorteil dieser Verbindungen sind ihre technischhervorragenden Eigenschaften. Es ist des weiteren bekannt, Ester aus Ölsäureoligomeren und/oder 12-Hydroxystearinsäure-oligomeren herzustellen. Diese Substanzen sind biologisch abbaubar, hydrolysestabil und können aus nachwachsenden Rohstoffen hergestellt werden.

Der vorliegenden Erfindung lag daher die Aufgabe zu Grunde, neue Esterverbindungen bereitzustellen, die den oben genannten Anforderungen genügen, d.h. sie müssen in Schmierstoffen einsetzbar sein und aus einfachen und leicht zugänglichen Ausgangsmaterialien herstellbar sein. Darüber hinaus sollte ein Syntheseverfahren zur Herstellung dieser Esterverbindungen bereitgestellt werden, mit dem eine hohe Ausbeute und eine hohe Selektivität erzielt werden, sowie eine einfache Aufreinigung möglich ist.

Diese Aufgabe wurde durch die Bereitstellung neuer Verfahren zur Herstellung von Esterverbindungen gelöst.

Die erfindungsgemäße Esterverbindung hat die nachfolgend gezeigte allgemeine Formel (I): 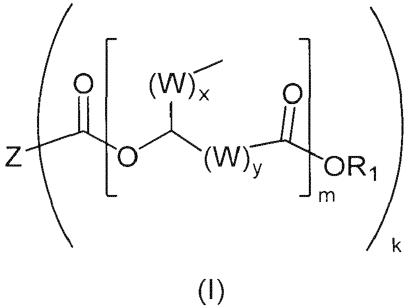 in der
der Rest Z ausgewählt wird aus dem Strukturelement einer Carbonsäure ohne Carboxyleinheiten, die mindestens ein sp3-hybridisiertes C-Atom mit einem oder keinem H-Atom, oder mindestens ein sp2-hybridisiertes C-Atom ohne H-Atome, oder mindestens ein Heteroatom in der Kette/Ring oder als Substituent enthält, ausgewählt aus der Gruppe bestehend aus hydrierter oder nicht hydrierter Dimersäure, hydrierter oder nicht hydrierter Trimersäure,
der Rest R¹ ausgewählt wird aus der Gruppe bestehend aus Wasserstoff, verzweigten oder unverzweigten C₁- bis C₃₀-Alkylresten, verzweigten oder unverzweigten C₁- bis C₃₀-Alkenylresten, C₇- bis C₃₀-Arylalkylresten und/oder C₁- bis C₃₀-Heteroarylalkylresten und C₄- bis C₃₀-Arylreste,
der Rest W ausgewählt wird aus der Gruppe bestehend aus -CH₂-und/oder -CH=CH-,
x ist eine ganze Zahl von 1 bis 20, vorzugsweise 1 bis 10,
y ist eine ganze Zahl von 1 bis 20, vorzugsweise 1 bis 10,
m ist eine ganze Zahl von 1 bis 10, vorzugsweise 1 bis 5,
k ist eine ganze Zahl von 2 bis 5.

Als Rest Z werden mehrwertige Carbonsäuren ausgewählt aus der Gruppe bestehend aus hydrierter oder nicht hydrierter Dimersäure, hydrierter oder nicht hydrierter Trimersäure eingesetzt.

Eine bevorzugte erfindungsgemäße Esterverbindung hat die nachfolgend gezeigte allgemeine Formel (II): 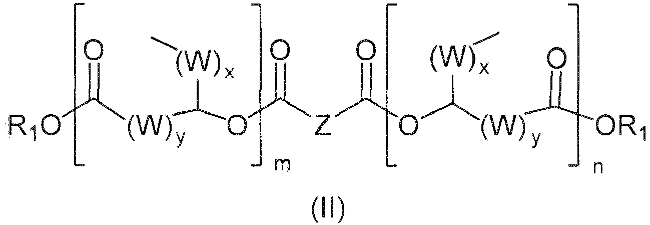 in der
der Rest Z ausgewählt wird aus dem Strukturelement einer Carbonsäure ohne Carboxyleinheiten, die mindestens ein sp3-hybridisiertes C-Atom mit einem oder keinem H-Atom, oder mindestens ein sp2-hybridisiertes C-Atom ohne H-Atome, oder mindestens ein Heteroatom in der Kette/Ring oder als Substituent enthält, ausgewählt aus der Gruppe bestehend aus hydrierter oder nicht hydrierter Dimersäure,
der Rest R¹ ausgewählt wird aus der Gruppe bestehend aus Wasserstoff, verzweigten oder unverzweigten C₁- bis C₃₀-Alkylresten, verzweigten oder unverzweigten C₁- bis C₃₀-Alkenylresten, C₇- bis C₃₀-Arylalkylresten und/oder C₁- bis C₃₀-Heteroarylalkylresten und C₄- bis C₃₀-Arylreste,
der Rest W ausgewählt wird aus der Gruppe bestehend aus -CH₂-und/oder -CH=CH-,
x ist eine ganze Zahl von 1 bis 20, vorzugsweise 1 bis 10,
y ist eine ganze Zahl von 1 bis 20, vorzugsweise 1 bis 10,
m ist eine ganze Zahl von 1 bis 10, vorzugsweise 1 bis 5,
n ist eine ganze Zahl von 1 bis 10, vorzugsweise 1 bis 5.

Als Rest Z werden mehrwertige Carbonsäuren ausgewählt aus der Gruppe bestehend aus hydrierter oder nicht hydrierter Dimersäure eingesetzt.

Die erfindungsgemäßen Esterverbindungen der allgemeinen Formel (I) und (II) können mit den nachfolgend beschriebenen Referenzverfahren A, Verfahren B sowie C synthetisiert werden.

Bei dem Referenzverfahren A werden Di-, Tri- oder höher funktionelle Carbonsäuren unter Einwirkung von Katalysatoren, wie Perchlorsäure mit ungesättigten Fettsäuren umgesetzt und anschließend mit Alkohol verestert.

Das Verfahren A zur Herstellung der erfindungsgemäßen Esterverbindung umfasst die Schritte:
(A) Zugabe eines Katalysators zu einer Di-, Tri- oder höher funktionellen Carbonsäure bei 50 bis 70°C für einen Zeitraum von 30 Minuten,
(B) Zugabe einer ungesättigten Fettsäure zu dem Gemisch über einen Zeitraum von 4 bis 8 Stunden, anschließend wird das Reaktionsgemisch bei 50 bis 70°C für 10 bis 14 Stunden gerührt,
(C) das erhaltene Zwischenprodukt wird mit Toluol/Ether verdünnt und mit Wasser mehrfach gewaschen,
(D) Veresterung des Zwischenprodukts mit einem Alkohol in Anwesenheit eines Katalysator bei 120 bis 150°C für 3 bis 5 Stunden unter Rühren, wobei entstehendes Wasser und das Restwasser/Ether in der organischen Phase z.B. mittels eines Wasserabscheiders unter reduziertem Druck entfernt werden,
(E) Waschen des Rohprodukts mit aq. NaHCOs-Lösung und Wasser,
(F) Trocknen über Na₂SO₄,
(G) Reinigung des Rohprodukts mittels eines Kurzwegverdampfers unter reduziertem Druck bei 190 bis 300°C.

Die ungesättigte Fettsäure wird bevorzugt ausgewählt aus der Gruppe bestehend aus Ölsäure und/oder Erucasäure.

Der Alkohol wird bevorzugt ausgewählt aus der Gruppe bestehend aus 2-Ethyl-hexan-1-ol und/oder 2-Propyl-heptan-1-ol und/oder 2-Hexyl-decan-1-ol und/oder 2-Octyldodecan-1-ol und/oder iso-Amylalkohol.

Als Katalysator wird in Reaktionsschritt (A) bevorzugt Perchlorsäure und in Reaktionsschritt (D) bevorzugt *p*-Toluolsulfonsäure eingesetzt.

Es sei noch angemerkt, dass die weiteren in den Verbindungen der Formeln (I) und (II) vorhandenen Doppelbindungen mit Carbonsäuren reagieren können.

Als alternative Syntheseroute werden gemäß Verfahren B langkettige Fettsäuren mit Hydroxygruppen unter Einwirkung von Katalysatoren, mit Di-, Tri oder höher funktionellen Carbonsäuren umgesetzt und anschließend verestert.

Dieses Verfahren B umfasst die Schritte:
(A) Umsetzung einer Di-, Tri- oder höher funktionellen Carbonsäure mit einer langkettigen Fettsäure mit Hydroxygruppe in Anwesenheit eines Katalysators bei 120 bis 150°C,
(B) Reduzierung des Drucks,
(C) Portionsweise oder kontinuierliche Zugabe der langkettigen Fettsäure mit Hydroxygruppe über einen Zeitraum von 5 bis 20 Stunden,
(D) Rühren des erhaltenen Reaktionsgemisches über 5 bis 20 Stunden unter reduziertem Druck und Entfernung des erhaltenen Wassers z.B. mittels eines Wasserabscheiders,
(E) Veresterung des Zwischenprodukts mit einem Alkohol bei 120 bis 150°C für 3 bis 5 Stunden unter Rühren,
(F) Waschen des Rohprodukts mit aq. NaHCOs-Lösung und Wasser,
(G) Trocknen über Na₂SO₄,
(H) Reinigung des Rohprodukts mittels eines Kurzwegverdampfers unter reduziertem Druck bei 190 bis 300°C.

Die langkettige Fettsäure mit Hydroxygruppen wird bevorzugt ausgewählt aus 12-Hydroxystearinsäure und/oder Ricinolsäure.

Der Alkohol wird bevorzugt ausgewählt aus der Gruppe bestehend aus 2-Ethyl-hexan-1-ol und/oder 2-Propyl-heptan-1-ol und/oder 2-Hexyl-decan-1-ol und/oder 2-Octyldodecan-1-ol und/oder iso-Amylalkohol.

Als Katalysator wird bevorzugt in Reaktionsschritt (A) p-Toluolsulfonsäure eingesetzt.

Ein weiteres bevorzugtes Syntheseverfahren ist das Verfahren C, bei dem eine langkettigen Fettsäure mit Hydroxygruppe unter Einwirkung von Katalysatoren mit einem Alkohol umgesetzt wird und das so erhaltene Zwischenprodukt mit einer Di-, Tri- oder höher funktionellen Carbonsäure verestert wird.

Das Verfahren C umfasst die Schritte:
(A) Umsetzung einer langkettigen Fettsäure mit Hydroxygruppe mit einem Alkohol in Anwesenheit eines Katalysators bei 60 bis 90°C,
(B) Reduzierung des Drucks,
(C) Rühren des erhaltenen Reaktionsgemisches über 6 bis 10 Stunden unter reduziertem Druck und Entfernung des erhaltenen Wassers,
(D) Entfernung des Lösungsmittels und überschüssigem Alkohol unter Vakuum,
(E) Umsetzung des Zwischenprodukts mit einer Di-, Tri- oder höher funktionellen Carbonsäure in Anwesenheit eines Katalysators bei 120 bis 160°C für 6 bis 10 Stunden unter Rühren und Entfernung des erhaltenen Wassers.
(F) Entfernen des Katalysators durch (Säure)-Waschen des Rohprodukts mit aq. NaHCOs-Lösung und Wasser oder durch Abfiltrieren von Träger gestützten Katalysatoren oder durch Verdampfen von flüchtigen Katalysatoren durch Anlegen eines Vakuums,
(G) Trocknen über geeigneten Trockenmitteln, wie beispielsweise Na₂SO₄,
(H) Reinigung des Rohprodukts unter reduziertem Druck bei 190 bis 300°C, beispielsweise mittels eines Kurzwegverdampfers.

Die langkettige Fettsäure mit Hydroxygruppen wird bevorzugt ausgewählt aus 12-Hydroxystearinsäure und/oder Ricinolsäure.

Der Alkohol wird bevorzugt ausgewählt aus der Gruppe bestehend aus 2-Ethyl-hexan-1-ol und/oder 2-Propyl-heptan-1-ol und/oder 2-Hexyl-decan-1-ol und/oder 2-Octyldodecan-1-ol und/oder iso-Amylalkohol.

Als Katalysator wird bevorzugt in Reaktionsschritt (A) und (E) *p*-Toluolsulfonsäure eingesetzt.

Alle Verfahren können teilweise oder komplett auch auf alternative Katalysatoren, z.B. Katalysatoren auf Enzymbasis umgestellt werden.

Beispiele für die Synthese nach Verfahren A, B und C:

### Referenzverfahren A

### Verwendung von Dimersäure und Ölsäure

Die Dimersäure wird durch nachfolgende Strukturformel in Verfahren A dargestellt.

Bei diesem Syntheseschema handelt es sich um eine Synthese einer Verbindung bzw. oligomeren Gemisches, bei der Perchlorsäure als Katalysator eingesetzt wird.

Die Reaktion ist nicht regioselektiv, so dass eine Verknüpfung an beiden olefinischen C-Atomen erfolgen kann (C9 oder C10; dargestellt durch die Kreise in der nachstehenden Abbildung). Des weiteren kann die Verknüpfung durch Umlagerungsreaktionen auch an anderen C-Atomen erfolgen, was in der nachstehenden Strukturformel nicht dargestellt ist.

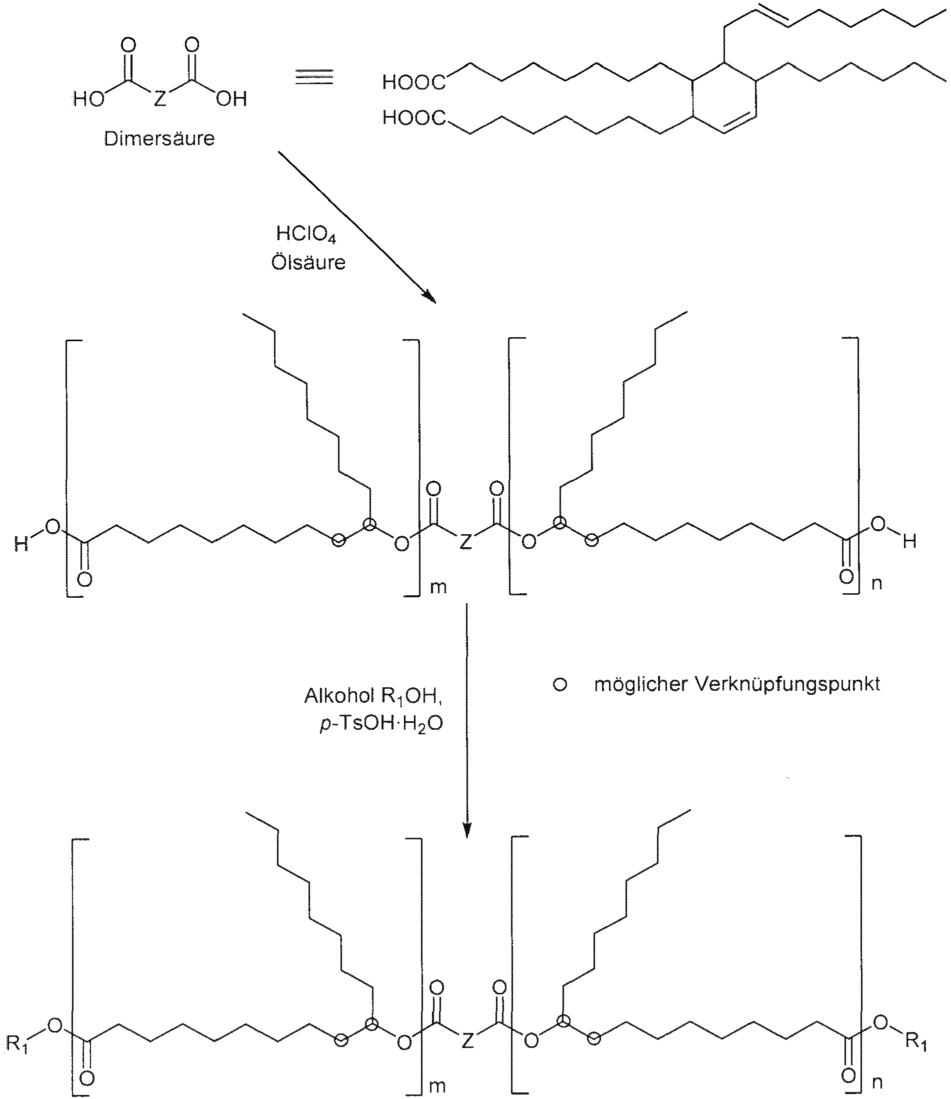

### Verfahren B

Verwendung einer mehrwertigen Carbonsäure und 12-Hydroxystearinsäure und *p-*Toluolsulfonsäure-monohydrat (*p*-TsOH·H₂O) als Katalysator.

Umlagerungsreaktionen spielen in diesem Verfahren keine Rolle, da die OH-Gruppe am C12-Kohlenstoff fixiert ist.

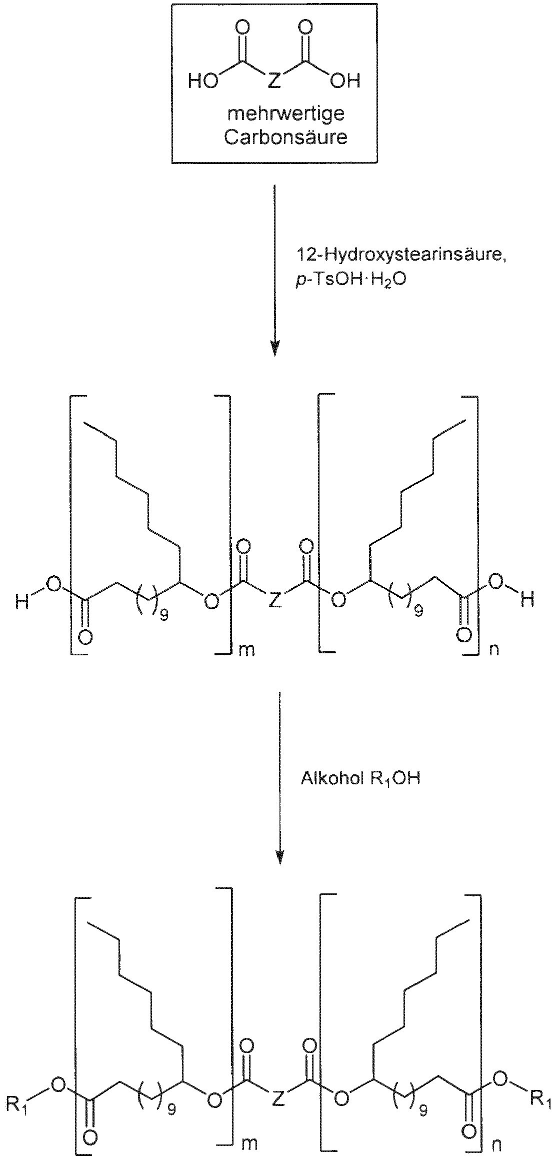

Bei dem Verfahren B wird als mehrwertige Carbonsäure eine Di,- Tri- oder höher funktionelle Carbonsäure eingesetzt, die bevorzugt ausgewählt wird aus der Gruppe bestehend aus hydrierter und nicht hydrierter Dimersäure, Trimersäure, 3,3'-Thiodipropionsäure, 2,2'-Thiodiessigsäure, Diglycolsäure, Itaconsäure, Phenylbernsteinsäure, Phthalsäureanhydrid, Cyclohexan-1,2-dicarbonsäureanhydrid, Cyclohexan-1,4-dicarbonsäure.

Bevorzugt wird 2-Ethylhexan-1-ol (als R₁OH in den obigen Abbildungen) zur Veresterung eingesetzt. Es können auch andere Alkohole eingesetzt werden.

So können iso-Amylalkohol und Guerbetalkolhol, wie beispielsweise 2-Hexyldecan-1-ol oder 2-Octyldodecan-1-ol, und 2-Propylheptan-1-ol verwendet werden.

### Verfahren C

Verwendung von 12-Hydroxystearinsäure und *p*-Toluolsulfonsäure-monohydrat (*p-*TsOH·H₂O) als Katalysator. 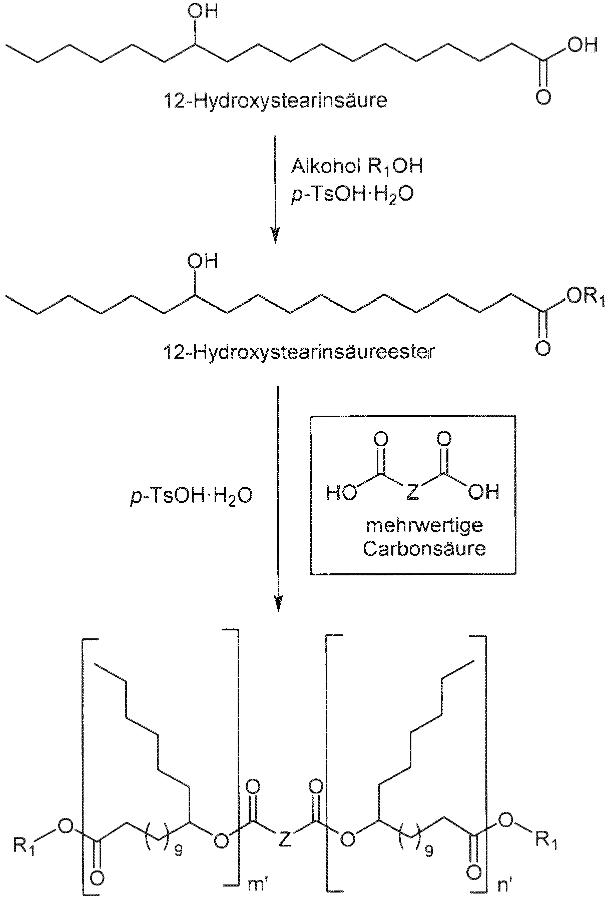 wobei m' ist eine ganze Zahl von 0 bis 10, vorzugsweise 0 bis 5, n' ist eine ganze Zahl von 0 bis 10, vorzugsweise 0 bis 5, Und m'+n' ≥ 1

Bevorzugt wird als R₁OH ein Alkohol ausgewählt aus der Gruppe bestehend aus i-Amylalkohol, 2-Ethylhexan-1-ol, 2-Propylheptan-1-ol, 2-Hexyldecan-1-ol, 2-Octyldodecan-1-ol eingesetzt.

Die mehrwertige Carbonsäure ist eine Di-, Tri-, bzw. höher funktionelle Carbonsäure, die wird vorzugsweise ausgewählt wird aus der Gruppe bestehend aus hydrierter oder nicht hydrierter Dimersäure.

Neben 12-Hydroxystearinsäure als langkettige Fettsäure mit Hydroxygruppen wird auch Ricinolsäure verwendet.

Die nach dem erfindungsgemäßen Verfahren hergestellten neuen Esterverbindungen werden in Schmierstoffzusammensetzungen eingesetzt.

Mit der erfindungsgemäßen Esterverbindung können Schmierstoffe bereitgestellt werden, die sowohl im Hochtemperaturbereich, im Marinebereich als auch im Lebensmittelbereich verwendet werden.

Neben der neuen Esterverbindung können die erfindungsgemäßen Schmiermittelzusammensetzungen weitere Grundölkomponenten auf der Basis von natürlichen Glyceridestern enthalten, vorzugsweise Sonnenblumenöl, Rapsöl oder Rüböl, Leinöl, Maisöl oder Maiskeimöl, Distelöl, Sojabohnenöl, Leinsamenöl, Erdnussöl, "Lesqueralle"-Öl, Palmöl, Olivenöl, in der monomeren, oligomeren und/oder polymerisierten Form oder Mischungen aus den genannten Ölen.

Auch können Ester, wie Trimethylolpropan- und Pentaerytritolester, sowie TMP-Komplexester, vollständig oder teilweise mit gesättigten und/oder einfach oder mehrfach ungesättigten Carbonsäuren der Kettenlänge von 6 bis 36 Kohlenstoffatomen verestert sind. Diese können linear oder verzweigt sein.

Des weiteren können Komplexester aus Dimersäuren, Dimersäureester, wie Ethylhexyldimerat, aliphatischen Carbonsäure- und Dicarbonsäureester sowie Phosphatester, Trimellith- und Pyromellithsäureester, Ether, Polyetherpolyole, sowie Perfluorpolyether, Alkyldiphenylether und Polyphenylether, Silikonöle, Polyglycole bestehend aus statistisch verteilten Polyoxyethylen- und/oder Polyoxypropyleneinheiten und/oder anderen Polyoxyalkylenbausteinen sowie weitere Glycolderivate, verwendet werden. Auch ist der Einsatz von Polyalphaolefinen und Metallocen katalysiert hergestellten Polyalphaolefinen sowie Alphaolefin-Copolymeren möglich.

Ebenso ist die Verwendung von polymeren Systemen, wie beispielsweise nicht hydrierte, teilhydrierte oder vollhydrierte Polyisobutylen oder einer Mischung daraus, Styrol- und Polystyrol sowie deren Derivate und/oder polymere Systeme basierend auf Acrylaten, Acetatpolymeren und Amiden, Polyethylenen, Polypropylenen, halogenierten Polypropylenen und/ oder Cycloalkanen, möglich.

Des weiteren können Mineralöle, wie z.B. Weißöl, alkylierte Diphenylether, alkylierte Naphthaline sowie Perfluoropolyether und Silikonöle eingesetzt werden.

Das Schmiermittel, das die erfindungsgemäße Esterverbindung der allgemeinen Formel (I) enthält, kann sowohl in Form eines Schmieröls als auch als Schmierfett eingesetzt werden.

Das Schmiermittel umfasst des weiteren Additive, die einzeln oder in Kombination eingesetzt werden und aus der Gruppe bestehend aus Korrosionsschutzadditiven, Antioxidanten, Verschleißschutzadditiven, UV-Stabilisatoren, anorganischen oder organischen Feststoffschmierstoffen, Pourpoint- und VI-Verbesserer, Polymere, Haftzusätze, Farbstoffe, Emulgatoren, Entschäumer und Festschmierstoffe ausgewählt werden und für die Formulierung eines Schmieröles bzw. Schmierfettes typisch sind.

Schmierfette können mit unterschiedlichen Verdickungsmitteln hergestellt werden. Eine mögliche Gruppe an Verdickungsmitteln sind Harnstoffe, die aus dem Reaktionsprodukt aus einem Diisocyanat, vorzugsweise 2,4-Diisocyanatotoluol, 2,6-Diisocyanatotoluol, 4,4`-Diisocyanatodiphenylmethan, 2,4'-Diisocyanatophenylmethan, 4,4'-Diisocyanatodi-phenyl, 4,4'-Diisocyanato-3-3'-dimethylphenyl, 4,4'-Diisocyanato-3,3'-dimethylphenylmethan, die einzeln oder in Kombination verwendet werden können, mit einem Amin der allgemeinen Formel R'₂-N-R, oder einem Diamin der allgemeinen Formel R'₂-N-R-NR'₂, wobei R ein Aryl-, Alkyl- oder Alkylenrest mit 2 bis 22 Kohlenstoffatomen ist und R' identisch oder verschieden ein Wasserstoff, ein Alkyl-, Alkylen- oder Arylrest ist, oder mit Gemischen aus Aminen und Diaminen, bestehen.

Weitere mögliche Verdickungsmittel können Al-Komplexseifen, Metall-Einfachseifen der Elemente der ersten und zweiten Hauptgruppe des Periodensystems, Metall-Komplexseifen der Elemente der ersten und zweiten Hauptgruppe des Periodensystems, Bentonite, Sulfonate, Silikate, Aerosil, Polyimide oder PTFE oder eine Mischung der vorgenannten Verdickungsmittel sein.

Um den gesetzlichen Bestimmungen bezüglich der Verwendung von Schmierstoffen zum Schmieren von Arbeitsgeräten für die Verarbeitung von Lebensmitteln zu entsprechen, ist es zweckmäßig, wenn die eingesetzten Additive und Verdickungsmittel eine H1-Klassifikation aufweisen.

Der Zusatz von Antioxidantien kann die Oxidation des erfindungsgemäßen Öls oder Fetts, insbesondere bei seinem Einsatz, verringern oder gar verhindern.

Die Antioxidantien werden ausgewählt aus der Gruppe bestehend aus aromatischen Diarylaminen, Phenolen, Thiophenolen, Phosphiten, butyliertem Hydroxytoluol, butyliertem Hydroxyanisol, Phenyl-alpha-naphthylaminen, Phenyl-betanaphthylaminen, octylierten/butylierten Diphenylaminen, di-alpha-Tocopherol, Benzolpropansäure und Mischungen dieser Komponenten.

Der erfindungsgemäße Schmierstoff kann Korrosionsschutzadditive, Metalldesaktivatoren oder lonen-Komplexbildner enthalten. Hierzu zählen Triazole, Imidazoline, N-Methylglycin (Sarcosin), Benzotriazolderivate, N,N-Bis(2-ethylhexyl)-ar-methyl-1 H-benzotriazol-1-methanamin; n-Methyl-N(1-oxo-9-octadecenyl)glycin, Gemisch aus Phosphorsäure und deren Mono-und Diisooctylester umgesetzt mit (C11-14)-Alkylaminen, Gemisch aus Phosphorsäure und Mono-und Diisooctylester umgesetzt mit tert.-Alkylamin und primären (C12-14)-Aminen, Dodekansäure, Triphenylphosphorthionat und Aminphosphate. Kommerziell sind derartige Additive unter den Namen: IRGAMET^{®} 39, IRGACOR^{®} DSS G, Amin O; SARKOSYL^{®} O (Ciba), COBRATEC^{®} 122, CUVAN^{®} 303, VANLUBE^{®} 9123, CI-426, CI-426EP, CI-429 und Cl-498 erhältlich.

Der erfindungsgemäße Schmierstoff kann Verschleißschutzadditive, Anti-Wear-Additive und Frictionmodifier enthalten.

Verschleißschutzadditive sind Amine, Aminphosphate, Phosphate, Thiophosphate, Phosphorthionate, Arylphosphat, Alkylierte Polysulfide, geschwefelte Aminverbindungen, geschwefelte Fettsäuremethylester, Naphthensäuren, Nanopartikel ausgewählt aus der Gruppe Al₂O₃, SiO₂, TiO₂, ZrO₂, WOs, Ta₂O₅, V₂O₅, CeO₂, Aluminiumtitanat, BN, MoSi2, SiC, Si₃N₄, TiC, TiN, ZrB₂, Tonminerale und/oder deren Gemische sowie thermisch stabile Carbonate und/oder Sulfate, und Mischungen dieser Komponenten. Zu den kommerziell erhältlichen Verschleißschutzadditiven gehören IRGALUBE^{®} TPPT, IRGALUBE^{®} 232, IRGALUBE^{®} 349, IRGALUBE^{®} 211 und ADDITIN^{®} RC3760 Liq 3960, FIRC-SHUN^{®} FG 1505 und FG 1506, NA-LUBE^{®} KR-015FG, LUBEBOND^{®}, FLUORO^{®} FG, SYNALOX^{®} 40-D, ACHESON^{®} FGA 1820 und ACHESON^{®} FGA 1810.

Der erfindungsgemäße Schmierstoff kann darüber hinaus Pourpoint- und Viskositätsverbesser und Haftzusätze enthalten.

Pourpoint- und Viskositätsverbesser werden ausgewählt aus der Gruppe bestehend aus linearen und/oder verzweigten alkylierten, acrylierten und aliphatischen Polymeren und Copolymeren sowie polymerisierten Fettsäureestern, sowie aus der Gruppe PlB (Polyisobutylene) und PB (Polybutene), die teilhydriert oder vollhydriert verwendet werden.

Der erfindungsgemäße Schmierstoff kann UV-Stabilisatoren enthalten.

UV-Stabilisatoren werden ausgewählt aus der Gruppe bestehend aus Stickstoffheterocyclen, substituierten Stickstoffheterocyclen, linear und verzweigten alkylierten, acylierten, aliphatischen Stickstoffheterocyclen, sowie deren Derivate.

Der erfindungsgemäße Schmierstoff kann Festschmierstoffe enthalten.

Festschmierstoffe sind z.B. PTFE, BN, Pyrophosphat, Zn-Oxid, Mg-Oxid, Pyrophosphate, Thiosulfate, Mg-Carbonat, Ca-Carbonat, Ca-Stearat, Zn-Sulfid, Mosulfid, W-sulfid, Sn-Sulfid, Graphite, Graphen, Nano-Tubes, SiO₂-Modifikationen oder eine Mischung daraus enthalten.

Der erfindungsgemäße Schmierstoff kann Emulgatoren enthalten.

Emulgatoren sind ausgewählt aus den Gruppen der verzweigten und/oder linearen ethoxylierten und/oder propoxylierten Alkohole und deren Salze wie beispielsweise Alkohole, C16-C18, ethoxyliert, propoxyliert, Polyglykole, Fettsäureester, Silikate, ionische Tenside wie z. B. Natriumsalze von Alkylsulfonsäuren, wobei die Ketten C14-17-Kohlenstoffe enthalten.

Der erfindungsgemäße Schmierstoff kann Entschäumer enthalten.

Entschäumer werden ausgewählt aus der Gruppe bestehend aus ethoxylierten und/oder propoxylierten Alkohole der Kettenlängen C10-C18, Mono- und Diglyceride von Speisefetten, Acrylate, propoxylierte und/oder ethoxylierte Alkylether (Polyglycole), Alkohole, Siloxane.

Die erfindungsgemäßen Schmiermittelzusammensetzungen auf Basis der Esterverbindung der allgemeinen Formeln (I) oder (II) werden im Marinebereich, im Bereich der Binnengewässer und bei Offshore-Anlagen, d.h. zur Schmierung von Ketten, Gleitlagern, Propellerrudern, Propellerwellen, Maschinenbauteilen und Anlagen, die im Marinebereich mit Salzwasser oder in Binnengewässern mit Wasser und wässrigen Medien in Berührung kommen, eingesetzt. Des weiteren finden sie Anwendung bei der Schmierung von Arbeitsgeräten in der lebensmittelverarbeitenden Industrie, als Hydrauliköl in der lebensmittelverarbeitenden Industrie, für Transportund Steuerketten, für Vorrichtungen für die Verarbeitung von Getreide, Mehl und Tierfutter, sowie in Backöfen. Außerdem werden sie zur Schmierung von Wälz- und Gleitlagern, Transport- und Steuerketten in der Fahrzeugtechnik, der Fördertechnik, dem Maschinenbau, der Bürotechnik sowie in industriellen Anlagen und Maschinen, in den Bereichen der Haushaltsmaschinen und der Unterhaltungselektronik eingesetzt. Darüber hinaus werden sie zur Schmierung von Kegelrad- und Stirnradgetrieben von Laufrollenlagern in Stranggießanlagen und Transportrollenlagern in Durchlauföfen und zur offenen Zahnkrankschmierung an Drehrohröfen, Rohrmühlen, Trommeln und Mischern, wie sie speziell in der Zement-, Kalk-, Gips-, Minen- und Chemieindustrie, eingesetzt.

Die erfindungsgemäßen Esterverbindungen und ihre Herstellung sowie ihre Verwendung in einer Schmierstoffzusammensetzung werden nun anhand der folgenden Beispiele erläutert.

### Beispiele

### Referenzbeispiel 1

### Synthese der Esterverbindung

### Referenzverfahren A:

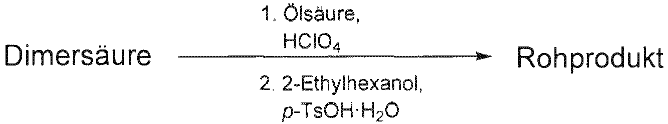

Dimersäure (100 g, Pripol 1013, CRODA) wurden mit HClO₄-Lösung (70% in Wasser, 60 g) versetzt und auf 60°C für 30 min erhitzt. Ölsäure (500 g, Radiacid 0137, OLEON) wurden bei 60 °C über einen Zeitraum von 4,5 Stunden zugetropft. Anschließend wurde das Reaktionsgemisch bei 60 °C für 13 h gerührt. Nach Abkühlung wurde das Produkt mit Toluol (300 ml) und Diethylether (100 ml) verdünnt und mit Wasser (7 x 600 ml) gewaschen. Zu der organischen Phase wurden 2-Ethylhexan-1-ol (250 g) und *p*-Toluolsulfonsäure-monohydrat (3 g) zugegeben und die Lösung bei 125°C für 5 h gerührt. Die entstehende Menge Wasser und das restliche Wasser/Ether in der organischen Phase wurden über einen Wasserabscheider entfernt. Es wurde reduzierter Druck angelegt, um den Destillationsprozess zu beschleunigen. Anschließend wurde das Reaktionsgemisch mit 4%-iger aq. NaHCOs-Lösung (2 x 400 ml) und Wasser (300 ml) gewaschen, über Na₂SO₄ getrocknet und unter reduziertem Druck eingeengt. Das Rohprodukt wurde mittels Kurzwegverdampfer fraktioniert destilliert.

Anstelle von 2-Ethylhexan-1-ol wurde auch 2-Propylheptan-1-ol, 2-Hexyldecan-1-ol (ISOFOL 16), 2-Octyldodecan-1-ol (ISOFOL 20) sowie iso-Amylalkohol verwendet.

Anstelle der Ölsäure kann Erucasäure eingesetzt werden.

### Verfahren B:

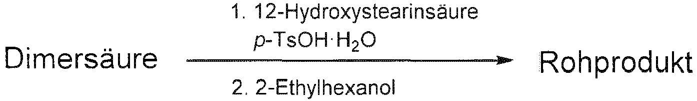

Dimersäure (90 g, Pripol 1013, CRODA) wurden mit 12-Hydroxystearinsäure (90 g, 12-HSA) und *p*-Toluolsulfonsäure-monohydrat (12,0 g) versetzt und auf 135 °C erhitzt. Zur Beschleunigung des Destillationsprozesses wurde der Druck reduziert und 5 mal nach jeweils 1,5 Stunden 12-HSA (90 g) zugegeben. Die Reaktionsmischung wurde bei 135 °C unter reduziertem Druck gerührt und die entstehende Menge Wasser (27,4 ml) über einen Wasserabscheider entfernt. Nach Zugabe letzter Portion von 12-HSA wurde die Reaktionsmischung bei 135 °C unter reduziertem Druck für 10 h gerührt. Anschließend wurde 2-Ethylhexan-1-ol (150 g) zugegeben und das Reaktionsgemisch bei 135 °C für 5 h gerührt. Die Reaktionsmischung wurde nach Abkühlung mit 4%-iger aq. NaHCOs-Lösung (500 ml) und Wasser (3 x 500 ml) gewaschen, über Na₂SO₄ getrocknet und unter reduziertem Druck eingeengt. Das Rohprodukt wurde mittels Kurzwegverdampfer fraktioniert destilliert.

Anstelle von 2-Ethylhexan-1-ol wurde auch 2-Propylheptan-1-ol, 2-Hexyldecan-1-ol (ISOFOL 16), 2-Octyldodecan-1-ol (ISOFOL 20) sowie iso-Amylalkohol verwendet.

Anstelle der 12-Hydroxystearinsäure kann Ricinolsäure eingesetzt werden.

### Verfahren C:

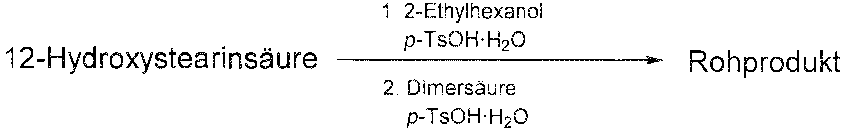

Eine Mischung von 12-Hydroxystearinsäure (500 g, 1,66 mol, 1,00 Äq.), 2-Ethylhexanol (542 g, 4,16 mol, 2,50 Äq.) und *p*-TsOH·H₂O (6,33 g, 33,3 mmol, 2 mol%) in Cyclohexan (600 ml) wurde bei 85 °C Ölbadtemperatur mit *Dean-Stark-App.* für 7,5 h gerührt. Leichtes Vakuum wurde angelegtund das durch Veresterung entstandene Wasser (insgesamt 30 ml) schneller überdestilliert wurde. Das Reaktionsgemisch wurde über Filterpapier filtriert, mit wässriger NaHCOs-Lösung (6 Gew.-%, 300ml) und Wasser (2 x 300 mL) gewaschen, über Na₂SO₄ getrocknet und unter reduziertem Druck eingeengt. Das Rohprodukt wurde unter Vakuum bei 0,1 mbar, T_{Ölbad}=110 °C abdestilliert und der Rückstand (670 g) wurde als Intermediat für nächsten Schritt aufbewahren.

Eine Mischung von Dimersäure (Pripol1013, Croda, 50 g), Intermediat von erstem Schritt (12-HSA-Ester, 104 g) und *p*-TsOH Monohydrat (2,5 g) in Toluol (150 ml) wurde bei 150 °C Ölbadtemperatur mit *Dean-Stark-App.* für 8 h gerührt. Das entstandene Wasser (insgesamt 3,6 ml) wurde überdestilliert. Das Reaktionsgemisch wurde mit Diethylether (50 ml) verdünnt, mit wässriger NaHCOs-Lösung (5 Gew.-%, 2 x 100 ml) und Wasser (2 x 100 ml) gewaschen, über Na₂SO₄ getrocknet und unter reduziertem Druck eingeengt. Das Rohprodukt wurde mittels Kurzwegverdampfer fraktioniert destilliert.

Anstelle von 2-Ethylhexan-1-ol wurde auch 2-Propylheptan-1-ol, 2-Hexyldecan-1-ol (ISOFOL 16), 2-Octyldodecan-1-ol (ISOFOL 20) sowie iso-Amylalkohol verwendet.

Anstelle der 12-Hydroxystearinsäure kann Ricinolsäure eingesetzt werden.

Die erhaltenen Rohprodukte wurden, wie bereits beschrieben, mit einem Kurzwegverdampfer (Modell VKL 70-4 FDRR-SKR-T der Firma VTA) unter Anlegen eines geeigneten Vakuums gereinigt und die Destillationsbedingungen mittels einer GPC-Analyse optimiert. Bei 1. Destillationsschritt bei 200°C wurde Lösungsmittel und nicht reagierter Alkohol entfernt. Bei der anschließenden 2. Destillation durfte die Temperatur 300°C nicht übersteigen, da bei dieser Temperatur die Esterpyrolyse einsetzt.

Für die folgenden neuen Esterverbindungen wurden die chemischen und physikalischen Eigenschaften geprüft, diese sind in Tabelle 1 gezeigt.

Verbindung (1) ist das Reaktionsprodukt aus Dimersäure/Ölsäure/2-Ethylhexan-1-ol, Verbindung (2) ist das Reaktionsprodukt aus Dimersäure/Ölsäure/ISOFOL 16, Verbindung (3) ist das Reaktionsprodukt aus Dimersäure/Ölsäure/ISOFOL 20, Verbindung (4) ist das Reaktionsprodukt aus Dimersäure/12-HSA/2-Ethylhexan-1-ol, Verbindung (5) ist das Reaktionsprodukt aus 12-HSA/2-Ethylhexan-1-ol/Dimersäure, wobei die Verbindungen (1) bis (3) nach Referenzverfahren A, die Verbindung (4) nach Verfahren B und die Verbindung (5) nach Verfahren C hergestellt wurden.

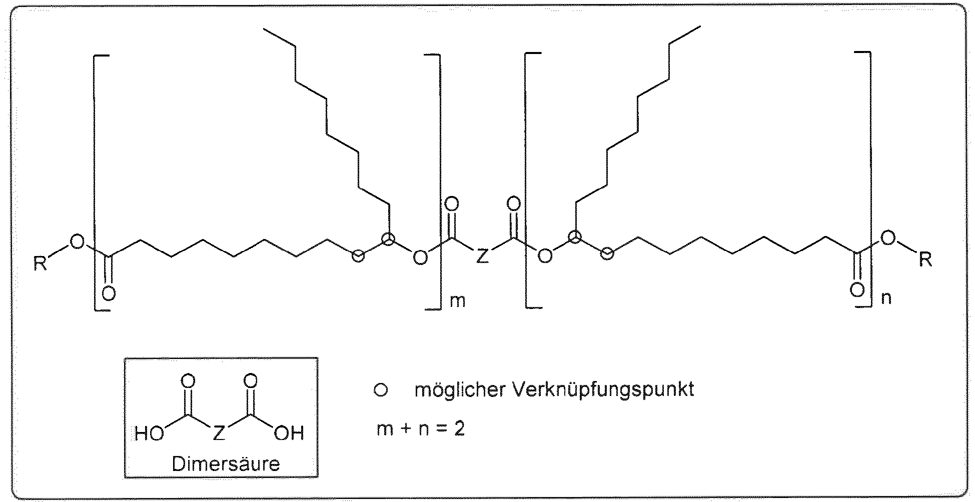

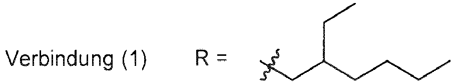

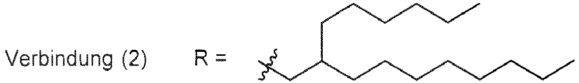

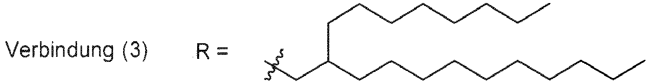

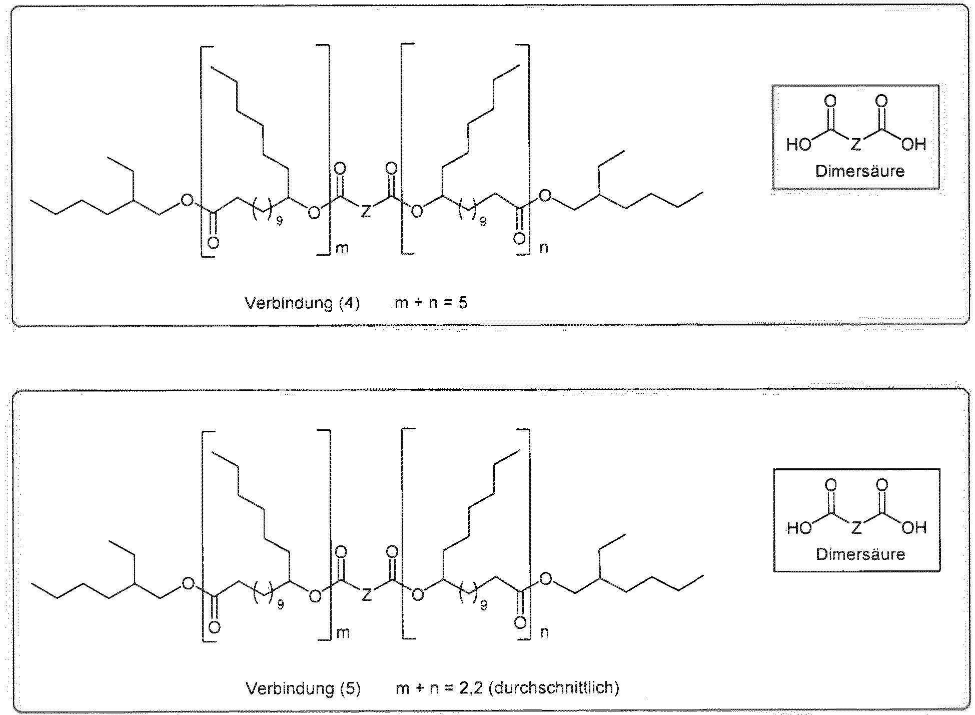

**Tabelle 1**

| Chemische und physikalische Eigenschaften der Verbindungen (1) bis (5) | | | | | | | |
|---|---|---|---|---|---|---|---|
| Parameter | Methode | Einheit | (1) | (2) | (3) | (4) | (5) |
| Aussehen | | | flüssig | flüssig | flüssig | flüssig | flüssig |
| | | | klar | klar | klar | klar | klar |
| | | | braun | braun | braun | braun | braun |
| Kin. Vis. 40°C | ASTM | mm²/s | 202 | 300 | 261 | 497 | 275 |
| Kin. Vis. 100°C | D 7042 | | 25 | 35 | 32 | 54 | 32 |
| VI | | | 157 | 160 | 163 | 174 | 159 |
| Neutralisationszahl | DIN 51558 | mg KOH/g | 1,3 | 1,6 | 1,1 | 0,65 | 0,55 |
| Pourpoint | DIN ISO 3016 | °C | -30 | -45 | -39 | -24 | -42 |
| biologische Abbaubarkeit | OECD 301 F | % | 50,9 | 50,2 | 60 | 66,8 | 45,6 |

**Tabelle 2**

| Vergleichsbeispiele der Verbindungen (A) bis (C) | | | | | |
|---|---|---|---|---|---|
| Parameter | Methode | Einheit | Ester A | Ester B | Ester C |
| biologische Abbaubarkeit | OECD 301 F | % | 30,2 | 25,0 | 34,2 |

| | | | | | |
|---|---|---|---|---|---|
| Ester A: Diester aus Dimersäure/2-Ethylhexan-1-ol, Ester B: Diester aus Dimersäure/*i*Amylalkohol, Ester C: Diester aus Dimersäure/ISOFOL 16. | | | | | |

Der Vergleich der Verbindungen (1) bis (3) zeigt, dass eine bessere biologische Abbaubarkeit erreicht wird, je größer der molekulare Anteil des Alkohols ist. Darüber hinaus wird aus Tabelle 2 deutlich, dass reine Diester (= Verbindungen A bis C) aus Dimersäure und den verwendeten Alkoholen nach OECD 301 F nicht biologisch abbaubar sind. Die biologische Abbaubarkeit des Esters kann durch entsprechende Modifikation mit Ölsäure bzw. 12-HSA (= Verbindungen (1) bis (4)) deutlich erhöht werden (Vergleich Tabelle 1 und 2).

Darüber hinaus hat sich gezeigt, dass die Verbindung (4), die gemäß dem Verfahren B hergestellt wurde, die besten chemischen und physikalischen Eigenschaften aufwies.

**Tabelle 3**

| Beverage Bottle Test ASTM D 2619 (Einheit: TAN [mg KOH/g], kin. Visk. [mm²/s]) | | | | | | | |
|---|---|---|---|---|---|---|---|
| Produkt | TAN wäss. Phase | TAN Öl vorher | TAN Öl nachher | Δ TAN Öl | Visk. Öl 40°C vorher | Visk. Öl 40°C nachher | Δ Visk. Ö140°C |
| (1) | 1,32 | 1,3 | 4,3 | 3 | 201 | 196 | -5 |
| (3) | 0,59 | 1,1 | 2,3 | 1,2 | 261 | 254 | -7 |
| (4) | 1,1 | 0,65 | 1,56 | 0,91 | 497 | 473 | -24 |
| Referenzester | 3 | 0,2 | 12,9 | 12,7 | 963,1 | 650,8 | -312,3 |

Referenzester: 1,1,1-Trimethylolpropan (TMP)-Ester gesättigt; biologisch abbaubar; ISO VG 1000,

Der Beverage Bottle Test wird verwendet um die Hydrolysebeständigkeit von Schmierstoffen bzw. Grundölen zu bestimmen. Tabelle 3 zeigt, dass die erfindungsgemäßen Produkte (1), (3) und (4) im Vergleich zum Referenzester sehr hydrolysestabil sind. Die Säurezahl und die kinematische Viskosität verändern sich deutlich weniger.

Weitere Esterverbindungen wurden synthetisiert aus unterschiedlichen mehrwertigen Carbonsäuren (s. Abbildung 1), Alkoholen R₁OH (s. Abbildung 2) und zwei Polymereinheiten (s. Abbildung 3), diese sind in Tabelle 4 (Verfahren B) und Tabelle 5 (Verfahren C) gezeigt. Die chemischen und physikalischen Eigenschaften sind in Tabelle 6 gezeigt.

| mehrwertige Carbonsäure: | | |
|---|---|---|
| Dimersäure **(S1)** | 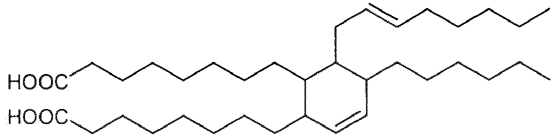 | |
| hyd. Dimersäure **(S2)** | 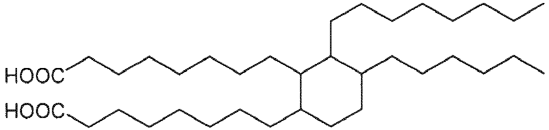 | |

| Trimersäure (**S3**, Struktur zu kompliziert) | | |
|---|---|---|
| 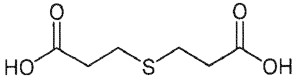 | 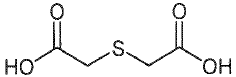 | 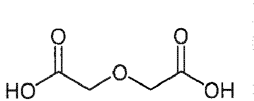 |
| 3,3'-Thiodipropionsäure **(S4)** | 2,2'-Thiodiessigsäure **(S5)** | Diglycolsäure **(S6)** |
| 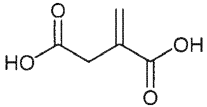 | 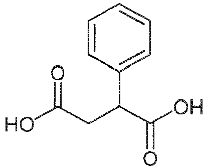 | 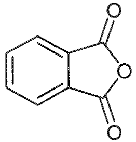 |
| Itaconsäure **(S7)** | Phenylbernsteinsäure **(S8)** | Phthalsäureanhydrid **(S9)** |
| 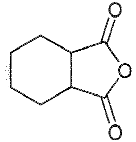 | 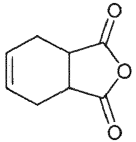 | |
| Cyclohexan-1,2-dicarbonsäureanhydrid (**S10**) | Cyclohexen-4,5-dicarbonsäureanhydrid **(S11)** | |

### Abbildung 1: Struktur der mehrwertigen Carbonsäuren Z(COOH)_{2/3}

| | | |
|---|---|---|
| Alkohole R₁OH: | | |
| 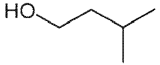 | 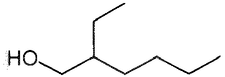 | 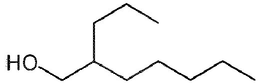 |
| *i*-Amylalkohol **(A1)** | 2-Ethylhexan-1-ol **(A2)** | 2-Propylheptan-1-ol **(A3)** |
| 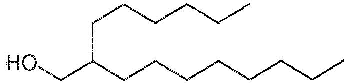 | 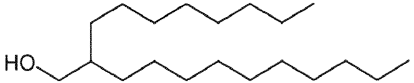 | |
| 2-Hexyldecan-1-ol (**A4**, ISOFOL 16) | 2-Octyldodecan-1-ol (**A5**, ISOFOL 20) | |

### Abbildung 2: Struktur der Alkohole R₁OH

| Polymereinheit im Klammer: |
|---|
| 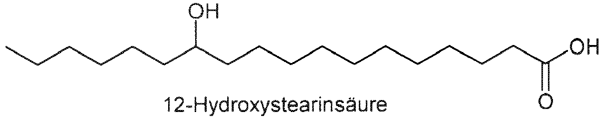 |
| 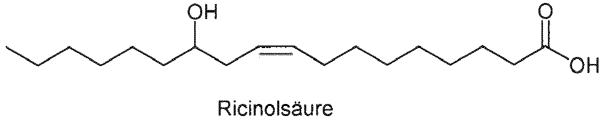 |

### Abbildung 3: Struktur der Polymereinheit 12-HSA und Ricinolsäure

**Tabelle 4**

| Esterverbindungen nach Verfahren B mit 12-HSA als Polymereinheit | | | | | |
|---|---|---|---|---|---|
| | Alkohole R₁OH (s. Abbildung 2) | | | | |
| | A1 | A2 | A3 | A4 | A5 |
| Dimersäure (S1) | (6) | (4) / (19)* | (20)* | (7) / (21)* | (8) |
| hyd. Dimersäure (52) | | (9) | | | |
| Trimersaure (S3) | | (10) | | | |
| 3,3'-Thiodipropionsäure (S4) | | (11) | | | |
| 2,2'-Thiodiessigsäure (55) | | (12) | | | |
| Diglycolsäure (S6) | | (13) | | | |
| Itaconsäure (S7) | | (14) | | | |
| Phenylbernsteinsäure (S8) | | (15) | | | |
| Phthalsäureanhydrid (S9) | | (16) | | | |
| Cyclohexan-1,2-dibarbonsäureanhydrid (S10) | | (17) | | | |
| Cyclohexen-4,5-dicarbonsaureanhydrid (S11) | | (18) | | | |

| | | | | | |
|---|---|---|---|---|---|
| * Bei Verbindung (19), (20) und (21) wurde Ricinolsäure statt 12-HSA verwendet. A1=*iso*-Amylalkohol, A2=2-Ethylhxan-1-ol, A3=2-Propylheptan-1-ol, A4=2-Hexyldecan-1-ol, A5=2-Octyldodecan-1 -ol | | | | | |

**Tabelle 5**

| Esterverbindungen nach Verfahren C mit 12-HSA als Polymereinheit | | | | | |
|---|---|---|---|---|---|
| | Alkohole R₁OH (s. Abbildung 2) | | | | |
| | A1 | A2 | A3 | A4 | A5 |
| Dimersäure (S1) | | (5) / (28)* | (22) | | |
| hyd. Dimersäure (52) | | | | | |
| Trimersäure (53) | | (23) / (29)* | | | |
| 3,3'-Thiodipropionsäure (54) | | (24) / (30)* | (25) | | |
| 2,2'-Thiodiessigsäure (S5) | | (26) / (31)* | (27) | | |
| Diglycolsaure (S6) | | | | | |

| | | | | | |
|---|---|---|---|---|---|
| * Bei Verbindung (28), (29), (30) und (31) wurde Ricinolsäure statt 12-HSA verwendet. A1=*iso*-Amylalkohol, A2=2-Ethylhxan-1-ol, A3=2-Propylheptan-1-ol, A4=2-Hexyldecan-1-ol, A5=2-Octyldodecan-1 -ol Das Mol-Verhältnis bleibt wie im Bespiel 1 nach Verfahren A, B und C. | | | | | |

**Tabelle 6**

| Chemische und physikalische Eigenschaften der Verbindungen (6) bis (31) | | | | | |
|---|---|---|---|---|---|
| Parameter | Kin. Vis. bei 40°C | Kin. Vis. bei 100°C | VI | Neutralisationszahl | Pourpoint |
| Methode | ASTM D7042 | | | DIN 51558 | DIN ISO 3016 |
| Einheit | mm²/s | mm²/s | | mg KOH/g | °C |
| Verbindung (6) | 473 | 53 | 177 | 0,2 | -21 |
| Verbindung (7) | 516 | 56 | 176 | 0,4 | -30 |
| Verbindung (8) | 578 | 63 | 181 | 0,7 | -30 |
| Verbindung (9) | 507 | 56 | 177 | 0,2 | -27 |
| Verbindung (10) | 568 | 61 | 178 | 0,6 | -24 |
| Verbindung (11) | 349 | 42 | 175 | 0,4 | -27 |
| Verbindung (12) | 322 | 38 | 170 | 0,3 | -24 |
| Verbindung (13) | 305 | 36 | 168 | 0,4 | -21 |
| Verbindung (14) | 318 | 33 | 169 | 0,4 | -24 |
| Verbindung (15) | 387 | 44 | 170 | 0,5 | -27 |
| Verbindung (16) | 452 | 50 | 173 | 0,5 | -24 |
| Verbindung (17) | 390 | 44 | 171 | 0,4 | -24 |
| Verbindung (18) | 403 | 46 | 172 | 0,4 | -24 |
| Verbindung (19) | 337 | 43 | 183 | 0,3 | -51 |
| Verbindung (20) | 376 | 46 | 181 | 0,5 | -51 |
| Verbindung (21) | 395 | 48 | 183 | 0,4 | -51 |
| Verbindung (22) | 306 | 34 | 155 | 1,0 | -48 |
| Verbindung (23) | 500 | 51 | 162 | 1,4 | -45 |
| Verbindung (24) | 108 | 16 | 158 | 1,0 | -42 |
| Verbindung (25) | 142 | 19 | 154 | 0,6 | -39 |
| Verbindung (26) | 107 | 16 | 153 | 1,5 | -42 |
| Verbindung (27) | 141 | 19 | 150 | 0,6 | -54 |
| Verbindung (28) | 230 | 29 | 164 | 0,5 | -51 |
| Verbindung (29) | 404 | 45 | 167 | 1,1 | -48 |
| Verbindung (30) | 106 | 16 | 167 | 0,7 | -60 |
| Verbindung (31) | 103 | 16 | 162 | 0,7 | -60 |

Die Tabelle zeigt, dass hervorragende Tieftemperatureigenschaften (siehe PP) erreicht werden können. Bei den Verbindungen (6), (7), (8), (9) und (10), (19) bis (22) sowie (28) handelt es sich um Dimersäuren, die Verbindungen (10 und (29) sind Trimersäuren. Hervorzuheben sind Verbindungen 19 bis 21, die trotz hoher Viskosität bei 40°C sehr niedrigen Pour Points zeigen. Erwähnenswert sind auch die durchgehend hohen Viskositäts indices (VI).

### Beispiel 2

### Herstellung eines Schmierfetts

Des weiteren wurde der neue Ester (Verbindung 1) mit 15% Li-Komplex-Verdicker verdickt und 4% Antioxidans zugegeben. Die genaue Zusammensetzung ist in Tabelle 7 angegeben.

Die Herstellung des Schmierfettes erfolgt nach dem Fachmann bekannten Vorgehen: Das Verdickungsmittel entsteht durch eine in situ-Reaktion der eingesetzten Reaktanten im Grundöl. Anschließend wird das Gemisch auf 150°C bis 210°C erhitzt, mehrere Stunden gerührt und wieder abgekühlt. Während des Abkühlprozesses werden bei ca. 60°C die notwendigen Additive hinzugegeben. Eine homogene Mischung des Fettes erhält man durch den abschließenden Homogenisierungsschritt über Walze, Kolloidmühle oder die Gaulin.

Die besondere Hydrolysestabilität des Grundöls ermöglicht die In situ Herstellung des Seifenverdickers.

**Tabelle 7**

| Komponente | Verbindung (1) | 12-HSA | Azelainsäure | LiOH·H₂O | Antioxidans |
|---|---|---|---|---|---|
| Anteil | 80,99% | 9,55% | 2,87% | 2,59% | 4,00% |

Das erhaltene Fett wurde auf seine chemischen und physikalischen Eigenschaften untersucht, die Ergebnisse sind in Tabelle 8 dargestellt. Es besitzt die Eigenschaften vom typischen Li-Komplex-Fett z.B. einen Tropfpunkt von>300 °C. Ergebnisse von Korrosionswirkung auf Kupfer, Wasserbeständigkeit und Geräuschtest sind sehr gut. Sehr gut/niedrig ist auch die Ölabscheidung bei hohen Temperaturen.

**Tabelle 8**

| Chemische und physikalische Eigenschaften des Schmierfetts aus Bsp. 2 | | | |
|---|---|---|---|
| Methodenname Norm | Bedingungen | Parameter | Schmierfett Bsp. 2 |
| Fließdruck DIN 51805 | Temperatur: -30 °C | Fließdruck (mbar) | 650 |
| | Temperatur: -35 °C | | 1525 |
| Konuspenetration DIN ISO 2137 | Anzahl Doppeltakte: 60 | Eindringtiefe (0,1 mm) | 283 |
| | Temperatur: 25 °C | | |
| | Konus: Standardkonus | | |
| Korrosionswirkung auf Kupfer DIN 51811 | Zeit: 24 h | Korrosionsgrad | 1a |
| | Temperatur: 150 °C | | |
| Neutralisationszahl DIN 51558 | Messzeitpunkt: nach Verseifung von 12-HSA und Azelainsäure | Neutralisationszahl (mg KOH/g) | 0,9630 |
| Ölabscheidung ASTM D 6184 | Zeit: 30 h | Ölabscheidung (%) | 3,02 |
| | Temperatur: 150 °C | | 2,83 |
| | | | 3,13 |
| Ölabscheidung ASTM D 6184 | Zeit: 30 h | Ölabscheidung (%) | 4,60 |
| | Temperatur: 180 °C | | 3,91 |
| Ölabscheidung DIN 51817 | Zeit: 168 h | Ölabscheidung (%) | 1,91 |
| | Temperatur: 40 °C | | 1,87 |
| Tropfpunkt DIN ISO 2176 | | Tropfpunkt (°C) | 344,7 |
| Verdampfungsverlust DIN 58397 T1 | Zeit: 24 h | Verdampfungsverlust (%) | 1,25 |
| | Temperatur: 150 °C | | 1,40 |
| Wasserbeständigkeit DIN 51807 Teil 1 | Zeit: 3 h | Bewertungsstufe | 0-90 |
| | Temperatur: 90 °C | | |
| Scherviskosität | original | (mPa·s) | 4991 |
| | Nach Lagerung bei 150 °C, 24 h | | 4310 |
| | | | 4270 |
| BeQuiet+ (SKF-Fettgeräuschprüfung) | | | GN 4 |

## Patentansprüche

1. Esterverbindung der allgemeinen Formel (**I**): 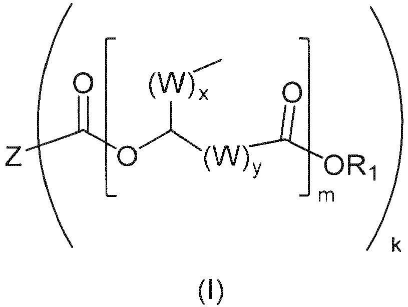 in der
der Rest Z ausgewählt wird aus dem Strukturelement einer Carbonsäure ohne Carboxyleinheiten, die mindestens ein sp3-hybridisiertes C-Atom mit einem oder keinem H-Atom, oder mindestens ein sp2-hybridisiertes C-Atom ohne H-Atome, oder mindestens ein Heteroatom in der Kette/Ring oder als Substituent enthält, ausgewählt aus der Gruppe bestehend aus hydrierter oder nicht hydrierter Dimersäure, hydrierter oder nicht hydrierter Trimersäure,
der Rest R¹ ausgewählt wird aus der Gruppe bestehend aus verzweigten oder unverzweigten C₁- bis C₃₀-Alkylresten, verzweigten oder unverzweigten C₁- bis C₃₀-Alkenylresten, C₇- bis C₃₀-Arylalkylresten und/oder C₁- bis C₃₀-Heteroarylalkylresten und C₄- bis C₃₀-Arylreste,
der Rest W ausgewählt wird aus der Gruppe bestehend aus -CH₂-und/oder - CH=CH-,
x ist eine ganze Zahl von 1 bis 20, vorzugsweise 1 bis 10,
y ist eine ganze Zahl von 1 bis 20, vorzugsweise 1 bis 10,
m ist eine ganze Zahl von 1 bis 10, vorzugsweise 1 bis 5,
k ist eine ganze Zahl von 2 bis 5.

2. Esterverbindung nach Anspruch 1 mit der allgemeinen Formel (II) 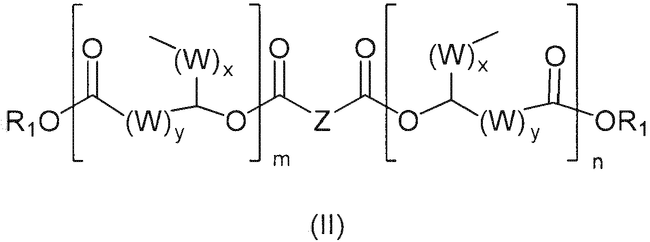 in der
der Rest Z ausgewählt wird aus dem Strukturelement einer Carbonsäure ohne Carboxyleinheiten, die mindestens ein sp3-hybridisiertes C-Atom mit einem oder keinem H-Atom, oder mindestens ein sp2-hybridisiertes C-Atom ohne H-Atome, oder mindestens ein Heteroatom in der Kette/Ring oder als Substituent enthält, ausgewählt aus der Gruppe bestehend aus hydrierter oder nicht hydrierter Dimersäure,
der Rest R¹ ausgewählt wird aus der Gruppe bestehend aus verzweigten oder unverzweigten C₁- bis C₃₀-Alkylresten, verzweigten oder unverzweigten C₁- bis C₃₀-Alkenylresten, C₇- bis C₃₀-Arylalkylresten und/oder C₁- bis C₃₀-Heteroarylalkylresten und C₄- bis C₃₀-Arylreste,
der Rest W ausgewählt wird aus der Gruppe bestehend aus -CH₂-und/oder - CH=CH-,
x ist eine ganze Zahl von 1 bis 20, vorzugsweise 1 bis 10,
y ist eine ganze Zahl von 1 bis 20, vorzugsweise 1 bis 10,
m ist eine ganze Zahl von 1 bis 10, vorzugsweise 1 bis 5,
n ist eine ganze Zahl von 1 bis 10, vorzugsweise 1 bis 5.

3. Verfahren zur Herstellung der Esterverbindung der allgemeinen Formeln (I) 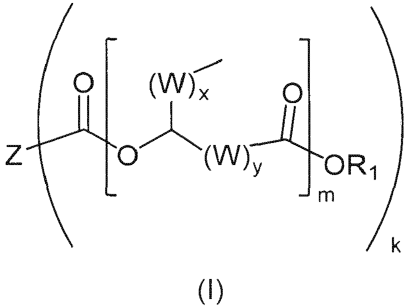 oder (II) 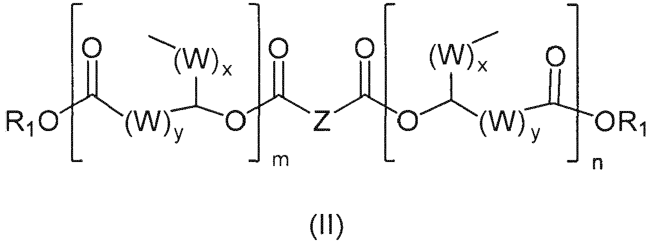 gemäß Anspruch 1 oder 2
umfassend die Schritte:
(A) Umsetzung einer Di-, Tri- oder höher funktionellen Carbonsäure mit einer langkettigen Fettsäure mit Hydroxygruppe in Anwesenheit eines Katalysators bei 120 bis 150°C,
(B) Reduzierung des Drucks,
(C) Portionsweise oder kontinuierliche Zugabe der langkettigen Fettsäure mit Hydroxygruppe über einen Zeitraum 5 bis 20 Stunden,
(D) Rühren des erhaltenen Reaktionsgemisches über 5 bis 20 Stunden unter reduziertem Druck und Entfernung des erhaltenen Wassers,
(E) Veresterung des Zwischenprodukts mit einem Alkohol bei 120 bis 150°C für 3 bis 5 Stunden unter Rühren,
(F) Waschen des Rohprodukts mit aq. NaHCOs-Lösung und Wasser,
(G) Trocknen über Na₂SO₄,
(H) Reinigung des Rohprodukts mittels eines Kurzwegverdampfers unter reduziertem Druck bei 190 bis 300°C.

4. Verfahren zur Herstellung von Esterverbindungen der allgemeinen Formeln (I) oder (II) nach Anspruch 3, bei dem die langkettige Fettsäure mit Hydroxygruppen ausgewählt wird aus 12-Hydroxystearinsäure und/oder Ricinolsäure; der Alkohol wird ausgewählt aus der Gruppe bestehend aus 2-Ethylhexan-1-ol und/oder 2-Propyl-heptan-1-ol und/oder 2-Hexyl-decan-1-ol und/oder2 -Octyldodecan-1-ol und/oder iso-Amylalkohol; der Katalysator in Reaktionsschritt (A) ist p-Toluolsulfonsäure.

5. Verfahren zur Herstellung von Esterverbindungen der allgemeinen Formeln (I) 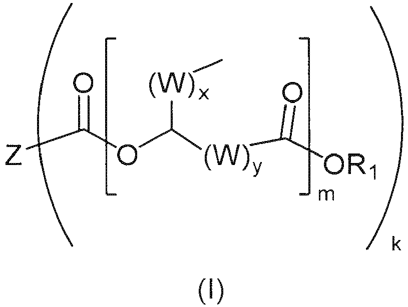 oder (II) 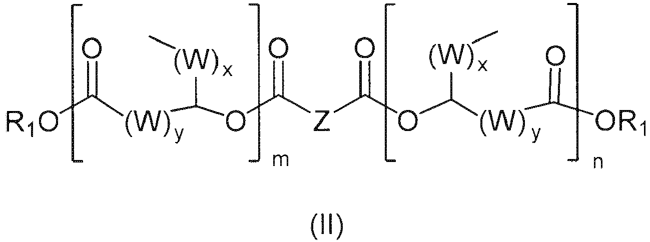 gemäß Anspruch 1 oder 2
umfassend die Schritte:
(A) Umsetzung einer langkettigen Fettsäure mit Hydroxygruppe mit einem Alkohol in Anwesenheit eines Katalysators bei 60 bis 90°C,
(B) Reduzierung des Drucks,
(C) Rühren des erhaltenen Reaktionsgemisches über 6 bis 10 Stunden unter reduziertem Druck und Entfernung des erhaltenen Wassers,
(D) Entfernung des Lösungsmittels und überschüssigem Alkohol unter Vakuum,
(E) Umsetzung des Zwischenprodukts mit einer Di-, Tri- oder höher funktionellen Carbonsäure in Anwesenheit eines Katalysators bei 120 bis 160°C für 6 bis 10 Stunden unter Rühren und Entfernung des erhaltenen Wassers.
(F) Entfernen des Katalysators durch (Säure)-Waschen des Rohprodukts mit aq. NaHCOs-Lösung und Wasser oder durch Abfiltrieren von Träger gestützten Katalysatoren oder durch Verdampfen von flüchtigen Katalysatoren durch Anlegen eines Vakuums,
(G) Trocknen über geeigneten Trockenmitteln, wie beispielsweise Na₂SO₄,
(H) Reinigung des Rohprodukts unter reduziertem Druck bei 190 bis 300°C, beispielsweise mittels eines Kurzwegverdampfers.

6. Verfahren zur Herstellung von Esterverbindungen der allgemeinen Formeln (I) oder (II) nach Anspruch 5 bei dem die langkettige Fettsäure mit Hydroxygruppen ausgewählt wird aus 12-Hydroxystearinsäure und/oder Ricinolsäure; der Alkohol wird ausgewählt aus der Gruppe bestehend aus 2-Ethylhexan-1-ol und/oder 2-Propyl-heptan-1-ol und/oder 2-Hexyl-decan-1-ol und/oder 2 - Octyldodecan-1-ol und/oder iso-Amylalkohol; der Katalysator in Reaktionsschritt (A) und (E) ist p-Toluolsulfonsäure.

7. Verwendung der Esterverbindung der allgemeinen Formeln (I) 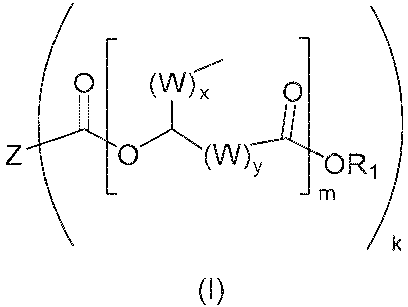 oder (II) 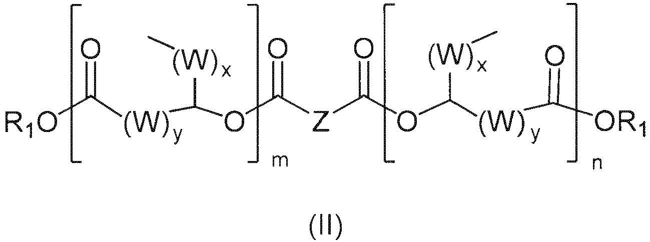 gemäß Anspruch 1 oder 2
in einer Schmiermittelzusammensetzung.

8. Verwendung der Esterverbindung der allgemeinen Formeln (I) oder (II) in einer Schmiermittelzusammensetzung nach Anspruch 7 im Marinebereich, zur Schmierung von Arbeitsgeräten in der lebensmittelverarbeitenden Industrie, zur Schmierung von Wälz- und Gleitlagern, Transport- und Steuerketten in der Fahrzeugtechnik, der Fördertechnik, dem Maschinenbau, der Bürotechnik zur Schmierung von Kegelrad- und Stirnradgetrieben von Laufrollenlagern in Stranggießanlagen und Transportrollenlagern in Durchlauföfen und zur offenen Zahnkrankschmierung an Drehrohröfen, Rohrmühlen, Trommeln und Mischern, wie sie speziell in der Zement-, Kalk-, Gips-, Minen- und Chemieindustrie.

9. Verwendung der Esterverbindung der allgemeinen Formeln (I) 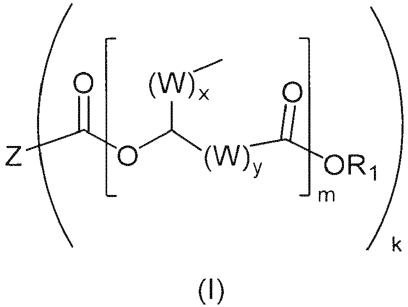 oder (II) 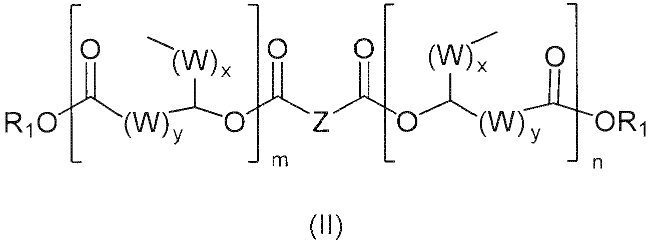 gemäß Anspruch 1 oder 2
in einer Schmiermittelzusammensetzung, die Schmiermittelzusammensetzung umfasst ein Grundöl, die Esterverbindung der allgemeinen Formeln (I) oder (II), Festschmierstoffe und Additive.

10. Verwendung nach Anspruch 9, wobei die Schmiermittelzusammensetzung des weiteren ein Verdickungsmittel umfasst.

11. Verwendung von Schmiermittelzusammensetzungen auf Basis der Esterverbindungen der allgemeinen Formeln (I) 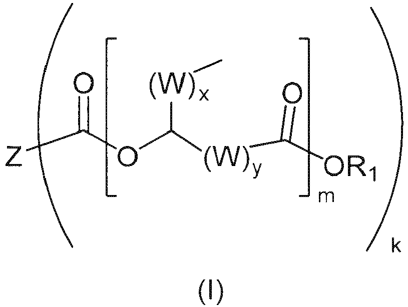 oder (II) 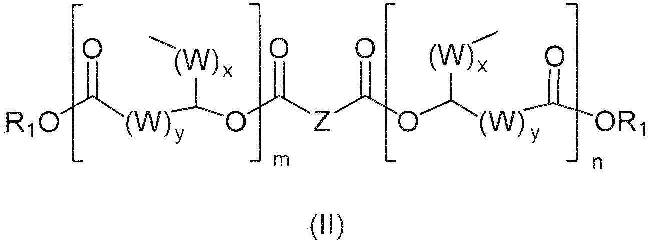 gemäß Anspruch 1 oder 2
im Marinebereich, zur Schmierung von Arbeitsgeräten in der lebensmittelverarbeitenden Industrie, zur Schmierung von Wälz- und Gleitlagern, Transport- und Steuerketten in der Fahrzeugtechnik, der Fördertechnik, dem Maschinenbau, der Bürotechnik zur Schmierung von Kegelrad- und Stirnradgetrieben von Laufrollenlagern in Stranggießanlagen und Transportrollenlagern in Durchlauföfen und zur offenen Zahnkrankschmierung an Drehrohröfen, Rohrmühlen, Trommeln und Mischern, wie sie speziell in der Zement-, Kalk-, Gips-, Minen- und Chemieindustrie.

## Claims

1. Ester compound of the general formula (I): 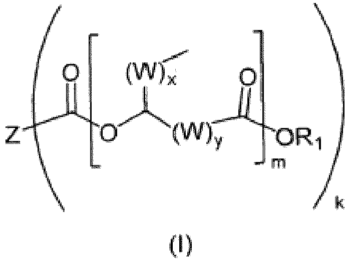 wherein
radical Z is selected from the structural element of a carboxylic acid without carboxyl units that contains at least one sp³ hybridized C atom with one or no H atom, or at least one sp² hybridized C atom without H atoms, or at least one heteroatom in the chain/ring or as a substituent, selected from the group consisting of hydrogenated or non-hydrogenated dimer acid, hydrogenated or non-hydrogenated trimer acid,
radical R₁ is selected from the group consisting of branched or unbranched C₁-C₃₀ alkyl radicals, branched or unbranched C₁-C₃₀ alkenyl radicals, C₇-C₃₀ arylalkyl radicals, and/or C₁-C₃₀ heteroarylalkyl radicals and C₄-C₃₀ aryl radicals,
radical W is selected from the group consisting of -CH₂- and/or -CH=CH-,
x is an integer between 1 and 20, preferably between 1 and 10,
y is an integer between 1 and 20, preferably between 1 and 10,
m is an integer between 1 and 10, preferably between 1 and 5,
k is an integer between 2 and 5.

2. Ester compound according to claim 1, of the general formula (II) 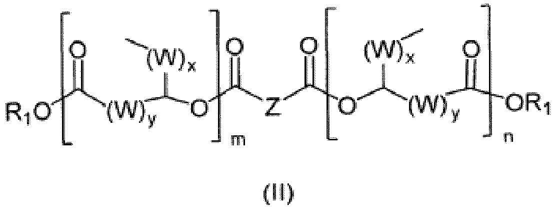 wherein
radical Z is selected from the structural element of a carboxylic acid without carboxyl units that contains at least one sp³ hybridized C atom with one or no H atom, or at least one sp² hybridized C atom without H atoms, or at least one heteroatom in the chain/ring or as a substituent, selected from the group consisting of hydrogenated or non-hydrogenated dimer acid,
radical R₁ is selected from the group consisting of branched or unbranched C₁-C₃₀ alkyl radicals, branched or unbranched C₁-C₃₀ alkenyl radicals, C₇-C₃₀ arylalkyl radicals, and/or C₁-C₃₀ heteroarylalkyl radicals and C₄-C₃₀ aryl radicals,
radical W is selected from the group consisting of -CH₂- and/or -CH=CH-,
x is an integer between 1 and 20, preferably between 1 and 10,
y is an integer between 1 and 20, preferably between 1 and 10,
m is an integer between 1 and 10, preferably between 1 and 5,
n is an integer between 1 and 10, preferably between 1 and 5.

3. Method for the preparation of the ester compound of the general formulae (I) 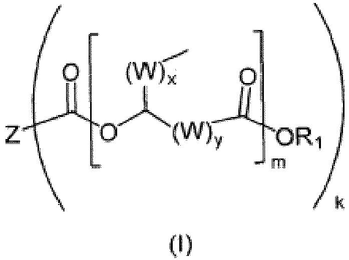 or (II) 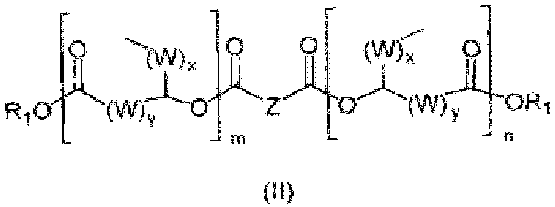 according to claim 1 or 2,
comprising the steps of:
(A) reacting a di-, tri- or higher functional carboxylic acid with a long-chain fatty acid with a hydroxyl group in the presence of a catalyst at between 120°C and 150°C,
(B) reducing the pressure,
(C) adding, in portions or continuously, the long-chain fatty acid with a hydroxyl group over a period of between 5 and 20 hours,
(D) stirring the reaction mixture obtained for between 5 and 20 hours under reduced pressure and while removing the water obtained,
(E) esterifying the intermediate product with an alcohol at between 120 °C and 150°C for 3 to 5 hours while stirring,
(F) washing the raw product with aq. NaHCQ₃ solution and water,
(G) drying over Na₂SO₄,
(H) purifying the raw product using a short path evaporator under reduced pressure at between 190°C and 300°C.

4. Method for the preparation of ester compounds of the general formulae (I) or (II) according to claim 3, wherein the long-chain fatty acid with hydroxyl groups is selected from 12-hydroxystearic acid and/or ricinoleic acid; the alcohol is selected from the group consisting of 2-ethylhexan-1-ol, and/or 2-propylheptan-1-ol, and/or 2-hexyldecan-1-ol, and/or 2-octyldodecan-1-ol, and/or isoamyl alcohol; the catalyst in reaction step (A) is p-toluene sulfonic acid.

5. Method for the preparation of ester compounds of the general formulae (I) 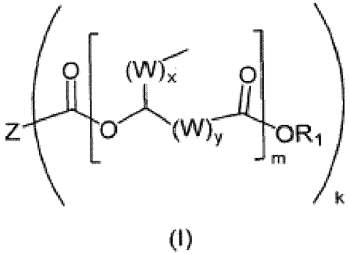 or (II) 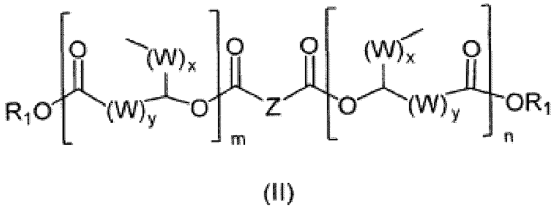 according to claim 1 or 2,
comprising the steps of:
(A) reacting a long-chain fatty acid with a hydroxyl group with an alcohol in the presence of a catalyst at between 60°C and 90°C,
(B) reducing the pressure,
(C) stirring the reaction mixture obtained for between 6 and 10 hours under reduced pressure and while removing the water obtained,
(D) removing the solvent and any excess alcohol under vacuum,
(E) reacting the intermediate product with a di-, tri- or higher functional carboxylic acid in the presence of a catalyst at between 120°C to 160°C for between 6 and 10 hours while stirring and removing the water obtained,
(F) removing the catalyst by (acid) washing of raw product with aq. NaHCO₃ solution and water, or by filtering off supported catalysts, or by evaporating volatile catalysts by applying a vacuum,
(G) drying over suitable drying agents, such as Na₂SO₄,
(H) purifying the raw product under reduced pressure at between190°C and 300°C, for example using a short-path evaporator.

6. Method for the preparation of ester compounds of the general formulae (1) or (II) according to claim 5, in which the long-chain fatty acid with hydroxyl groups is selected from 12-hydroxystearic acid and/or ricinoleic acid; the alcohol is selected from the group consisting of 2-ethylhexan-1-ol, and/or 2-propylheptan-1-ol, and/or 2-hexyldecan-1-ol, and/or 2-octyldodecan-1-ol, and/or isoamyl alcohol; the catalyst in reaction steps (A) and (E) is p-toluene sulfonic acid.

7. Use of the ester compound of the general formulae (I) 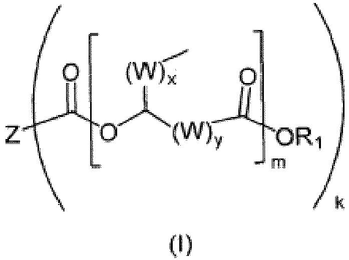 or (II) 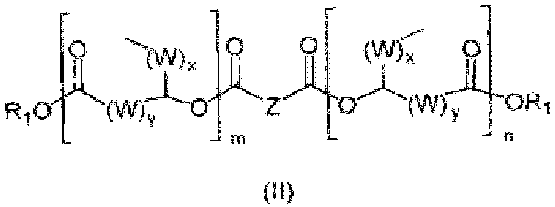 according to claim 1 or 2 in a lubricant composition.

8. Use of the ester compound of the general formulae (I) or (II) in a lubricant composition according to claim 7 in the marine sector, for lubricating tools in the food processing industry, for lubricating rolling and plain bearings, transport and control chains in automotive manufacturing, materials handling equipment, mechanical engineering, office equipment for lubricating bevel gears and spur gears of idler roller bearings in continuous casting plants and transport roller bearings in continuous furnaces and for lubricating open gear rings in rotary kilns, tube mills, drums and mixers, as are used especially in the cement, lime, gypsum, mining and chemical industries.

9. Use of the ester compound of the general formulae (I) 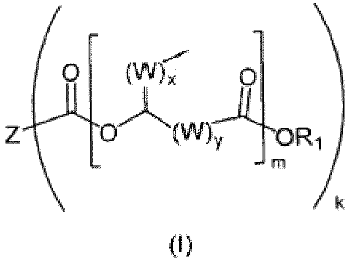 or (II) 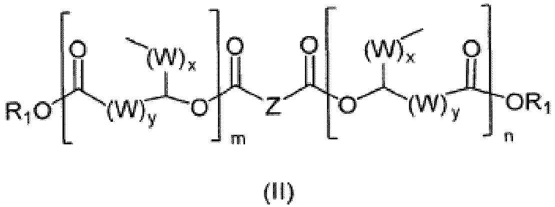 according to claim 1 or 2 in a lubricant composition, which lubricant composition comprises a base oil, the ester compound of the general formulae (I) or (II), solid lubricants and additives.

10. Use according to claim 9, wherein the lubricant composition further comprises a thickening agent.

11. Use of lubricant compositions based on the ester compounds of the general formulae (I) 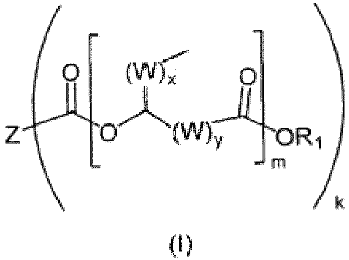 or (II) 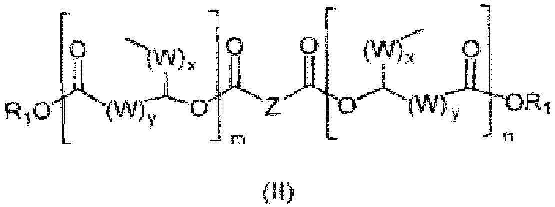 according to claim 1 or 2 in the marine sector, for lubricating tools in the food processing industry, for lubricating rolling and plain bearings, transport and control chains in automotive manufacturing, materials handling equipment, mechanical engineering, office equipment for lubricating bevel gears and spur gears of idler roller bearings in continuous casting plants and transport roller bearings in continuous furnaces and for lubricating open gear rings in rotary kilns, tube mills, drums and mixers, as are used especially in the cement, lime, gypsum, mining and chemical industries.

## Revendications

1. Composé ester de formule générale (I) : 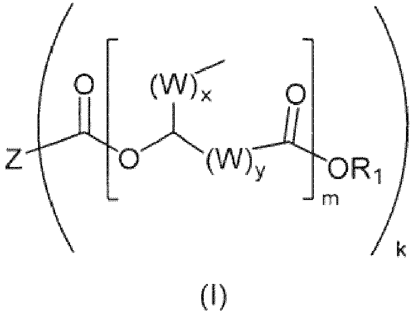 dans laquelle
le résidu Z est choisi à partir de l'élément structural d'un acide carboxylique sans unités carboxyle, qui contient au moins un atome de C hybridé en sp3 avec un atome de H ou aucun, ou au moins un atome de C hybridé en sp2 sans atomes de H, ou au moins un hétéroatome dans la chaîne/le cycle ou comme substituant, choisi dans le groupe constitué d'acide dimérique hydraté ou non hydraté, d'acide trimérique hydraté ou non hydraté,
le résidu R¹ est choisi dans le groupe constitué de résidus alkyle en C₁ à C₃₀ ramifiés ou non ramifiés, de résidus alcényle en C₁ à C₃₀ ramifiés ou non ramifiés, de résidus arylalkyle en C₇ à C₃₀ et/ou de résidus hétéroarylalkyle en C₁ à C₃₀ et de résidus aryle en C₄ à C₃₀,
le résidu W est choisi dans le groupe constitué de -CH₂-et/ou -CH=CH-,
x est un nombre entier de 1 à 20, de préférence de 1 à 10,
y est un nombre entier de 1 à 20, de préférence de 1 à 10,
m est un nombre entier de 1 à 10, de préférence de 1 à 5,
k est un nombre entier de 2 à 5.

2. Composé ester selon la revendication 1 de formule générale (II) 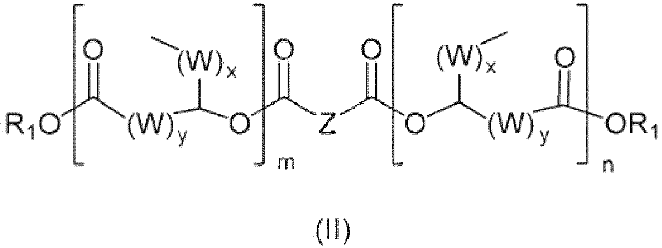 dans laquelle
le résidu Z est choisi à partir de l'élément structural d'un acide carboxylique sans unités carboxyle, qui contient au moins un atome de C hybridé en sp3 avec un atome de H ou aucun, ou au moins un atome de C hybridé en sp2 sans atomes de H, ou au moins un hétéroatome dans la chaîne/le cycle ou comme substituant, choisi dans le groupe constitué d'acide dimérique hydraté ou non hydraté,
le résidu R¹ est choisi dans le groupe constitué de résidus alkyle en C₁ à C₃₀ ramifiés ou non ramifiés, de résidus alcényle en C₁ à C₃₀ ramifiés ou non ramifiés, de résidus arylalkyle en C₇ à C₃₀ et/ou de résidus hétéroarylalkyle en C₁ à C₃₀ et de résidus aryle en C₄ à C₃₀,
le résidu W est choisi dans le groupe constitué de -CH₂-et/ou -CH=CH-,
x est un nombre entier de 1 à 20, de préférence de 1 à 10,
y est un nombre entier de 1 à 20, de préférence de 1 à 10,
m est un nombre entier de 1 à 10, de préférence de 1 à 5,
n est un nombre entier de 1 à 10, de préférence de 1 à 5.

3. Procédé de fabrication du composé ester de formules générales (I) 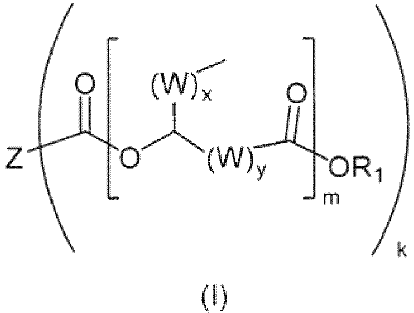 ou (II) 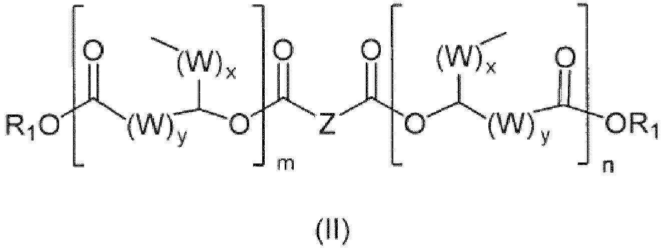 selon la revendication 1 ou 2
comprenant les étapes de :
(A) conversion d'un acide carboxylique difonctionnel, trifonctionnel ou de fonction supérieure avec un acide gras à longue chaîne pourvu de groupe hydroxy en présence d'un catalyseur à une température de 120 à 150 °C,
(B) réduction de la pression,
(C) addition par portion ou en continu de l'acide gras à longue chaîne pourvu de groupe hydroxy pendant une durée de 5 à 20 heures,
(D) agitation du mélange de réaction obtenu pendant 5 à 20 heures sous pression réduite et élimination de l'eau obtenue,
(E) estérification du produit intermédiaire avec un alcool à une température de 120 à 150°C pendant 3 à 5 heures sous agitation,
(F) lavage du produit brut avec une solution aqueuse de NaHCO₃ et de l'eau,
(G) séchage sur Na₂SO₄,
(H) purification du produit brut au moyen d'un évaporateur direct sous pression réduite à une température de 190 à 300 °C.

4. Procédé de fabrication de composés esters de formules générales (I) ou (II) selon la revendication 3, dans lequel l'acide gras à longue chaîne pourvu de groupes hydroxy est choisi parmi l'acide 12-hydroxystéarique et/ou l'acide ricinolique ; l'alcool est choisi dans le groupe constitué du 2-éthylhexan-1-ol et/ou du 2-propyl-heptan-1-ol et/ou du 2-hexyldécan-1-ol et/ou du 2-octyldodécan-1-ol et/ou de l'alcool isoamylique ; le catalyseur à l'étape de réaction (A) est l'acide *p*-toluènesulfonique.

5. Procédé de fabrication des composés esters de formules générales (I) 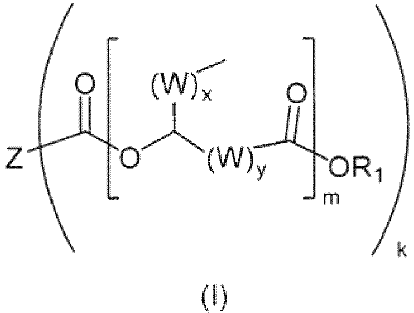 ou (II) 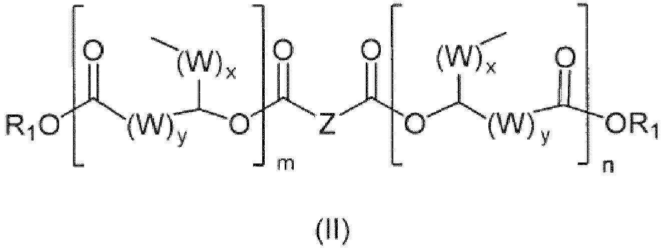 selon la revendication 1 ou 2
comprenant les étapes de :
(A) conversion d'un acide gras à longue chaîne pourvu de groupe hydroxy avec un alcool en présence d'un catalyseur à une température de 60 à 90 °C,
(B) réduction de la pression,
(C) agitation du mélange de réaction obtenu pendant 6 à 10 heures sous pression réduite et élimination de l'eau obtenue,
(D) élimination du solvant et de l'alcool excédentaire sous vide,
(E) conversion du produit intermédiaire avec un acide carboxylique bifonctionnel, trifonctionnel ou de fonction supérieure en présence d'un catalyseur à une température de 120 à 160°C pendant 6 à 10 heures sous agitation et élimination de l'eau obtenue,
(F) élimination du catalyseur par lavage (à l'acide) du produit brut avec une solution aqueuse de NaHCO₃ et de l'eau ou par filtration des catalyseurs supportés par support ou par évaporation des catalyseurs volatils par application d'un vide,
(G) séchage sur des agents dessiccatifs appropriés, comme par exemple Na₂SO₄,
(H) purification du produit brut sous pression réduite à une température de 190 à 300 °C, par exemple au moyen d'un évaporateur direct.

6. Procédé de fabrication de composés esters de formules générales (I) ou (II) selon la revendication 5 dans lequel l'acide gras à longue chaîne pourvu de groupes hydroxy est choisi parmi l'acide 12-hydroxystéarique et/ou l'acide ricinolique ; l'alcool est choisi dans le groupe constitué du 2-éthylhexan-1-ol et/ou du 2-propyl-heptan-1-ol et/ou du 2-hexyldécan-1-ol et/ou du 2-octyldodécan-1-ol et/ou de l'alcool isoamylique ; le catalyseur aux étapes de réaction (A) et (E) est l'acide *p*-toluènesulfonique.

7. Utilisation du composé ester de formules générales (I) 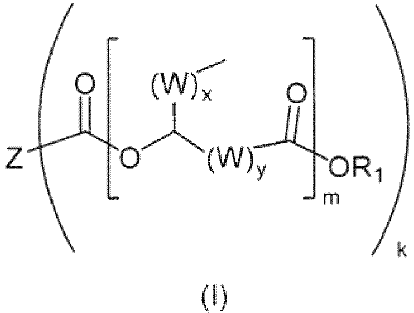 ou (II) 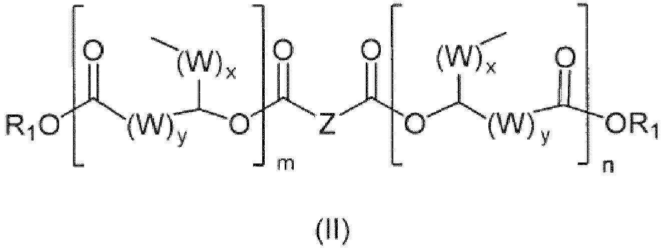 selon la revendication 1 ou 2
dans une composition lubrifiante.

8. Utilisation du composé ester de formules générales (I) ou (II) dans une composition lubrifiante selon la revendication 7 dans le domaine de la marine, pour la lubrification d'outils de travail dans l'industrie de transformation des produits alimentaires, pour la lubrification de paliers à roulement et paliers lisses, de chaînes de transport et de commande dans le génie automobile, dans la manutention, dans le génie mécanique, dans l'équipement bureautique pour la lubrification d'engrenages à roue conique et à roue droite de paliers à roulettes dans des installations de coulée continue et des paliers à rouleaux transporteurs dans des fours continus et pour la lubrification ouverte de roues dentées sur des fours tubulaires rotatifs, des broyeurs tubulaires, des tambours et des mélangeurs, tels qu'ils se présentent spécifiquement dans l'industrie du ciment, de la chaux, du plâtre, des mines et de la chimie.

9. Utilisation du composé ester de formules générales (I) 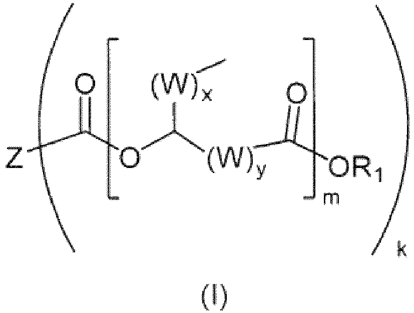 ou (II) 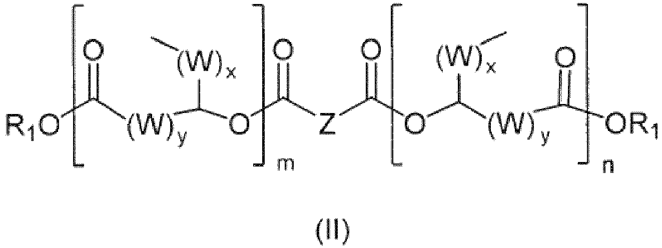 selon la revendication 1 ou 2
dans une composition lubrifiante, la composition lubrifiante comprenant une huile de base, le composé ester de formules générales (I) ou (II), des lubrifiants solides et des additifs.

10. Utilisation selon la revendication 9, dans laquelle la composition lubrifiante comprend en outre un agent épaississant.

11. Utilisation de compositions lubrifiantes à base des composés esters de formules générales (I) 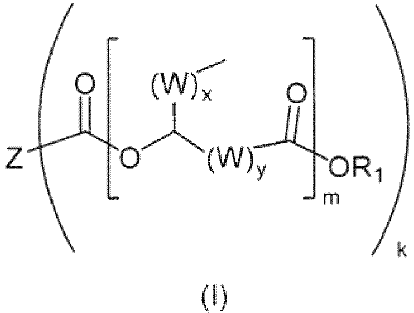 ou (II) 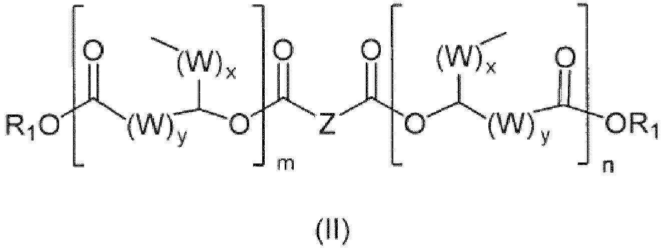 selon la revendication 1 ou 2
dans le domaine de la marine, pour la lubrification d'outils de travail dans l'industrie de transformation des produits alimentaires, pour la lubrification de paliers à roulement et paliers lisses, de chaînes de transport et de commande dans le génie automobile, dans la manutention, dans le génie mécanique, dans l'équipement bureautique pour la lubrification d'engrenages à roue conique et à roue droite de paliers à roulettes dans des installations de coulée continue et des paliers à rouleaux transporteurs dans des fours continus et pour la lubrification ouverte de roues dentées sur des fours tubulaires rotatifs, des broyeurs tubulaires, des tambours et des mélangeurs, tels qu'ils se présentent spécifiquement dans l'industrie du ciment, de la chaux, du plâtre, des mines et de la chimie.
